(19) Europäisches Patentamt
European Patent Office
Office européen des brevets

(11) Veröffentlichungsnummer: **0 059 389**
**B1**

(12) **EUROPÄISCHE PATENTSCHRIFT**

(45) Veröffentlichungstag der Patentschrift: **12.12.84**

(51) Int. Cl.³: **C 07 D 487/04,** A 61 K 31/55 // C07D495/14 ,(C07D487/04, 243/00, 235/00)

(21) Anmeldenummer: **82101271.3**

(22) Anmeldetag: **19.02.82**

(54) **Imidazodiazepine, Verfahren zu ihrer Herstellung, sowie diese enthaltende Arzneimittel.**

(30) Priorität: **27.02.81 CH 1340/81**

(43) Veröffentlichungstag der Anmeldung: **08.09.82 Patentblatt 82/36**

(45) Bekanntmachung des Hinweises auf die Patenterteilung: **12.12.84 Patentblatt 84/50**

(84) Benannte Vertragsstaaten: **AT BE CH DE FR IT LI LU NL SE**

(56) Entgegenhaltungen:
**EP - A - 0 027 214**
**DE - A - 2 609 486**
**DE - A - 2 813 549**

(73) Patentinhaber: **F. HOFFMANN-LA ROCHE & CO. Aktiengesellschaft, CH-4002 Basel (CH)**

(72) Erfinder: **Hunkeler, Walter, Dr., Im Stigler 32, CH-4465 Magden (CH)**
Erfinder: **Kyburz, Emilio, Dr., Unterer Rebbergweg 127, CH-4153 Reinach (CH)**

(74) Vertreter: **Lederer, Franz, Dr. et al, Patentanwälte Dr. Lederer Franz Meyer-Roxlau Reiner F. Lucile-Grahn-Strasse 22, D-8000 München 80 (DE)**

**EP 0 059 389 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die vorliegende Erfindung betrifft Imidazodiazepine. Im speziellen betrifft sie Imidazodiazepine der allgemeinen Formel

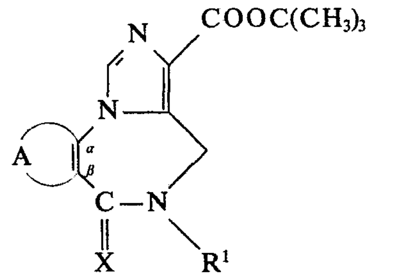

(I)

worin A zusammen mit den beiden mit $\alpha$ und $\beta$ bezeichneten Kohlenstoffatomen die Gruppe

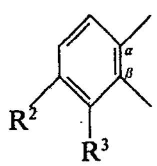

oder

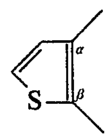

(a) (b)

X ein Sauerstoff- oder Schwefelatom und $R^1$ Wasserstoff oder niederes Alkyl bedeuten, wie im folgenden angegeben, und entweder $R^2$ Wasserstoff, Trifluormethyl, Halogen, Cyano oder Nitro und $R^3$ Wasserstoff bedeuten, oder $R^2$ Wasserstoff und $R^3$ Trifluormethyl, Halogen, Cyano, Nitro oder Alkyl bedeuten, wie im folgenden angegeben,
und pharmazeutisch annehmbare Säureadditionssalze davon.

Diese Verbindungen sind neu; sie zeichnen sich durch wertvolle pharmakodynamische Eigenschaften aus und können bei der Bekämpfung bzw. Verhütung von Krankheiten verwendet werden.

Gegenstand der vorliegenden Erfindung sind Verbindungen der allgemeinen Formel I und pharmazeutisch annehmbare Säureadditionssalze davon als solche und als pharmazeutische Wirkstoffe, die Herstellung dieser Verbindungen und Zwischenprodukte für die Herstellung dieser Verbindungen, ferner Arzneimittel, enthaltend eine Verbindung der allgemeinen Formel I oder ein pharmazeutisch annehmbares Säureadditionssalz davon, und die Herstellung solcher Arzneimittel.

Der Ausdruck »niederes Alkyl« bezeichnet gesättigte Kohlenwasserstoffreste, welche geradkettig oder verzweigt sein können, mit höchstens 7, vorzugsweise höchstens 4 Kohlenstoffatomen, wie Methyl, Äthyl, n-Propyl, Isopropyl, n-Butyl, s-Butyl, t-Butyl und dergleichen. Der Ausdruck »Halogen« bedeutet die vier Formen Fluor, Chlor, Brom und Jod.

Das Symbol A bedeutet vorzugsweise die Gruppe (a), wobei vorzugsweise entweder $R^2$ Wasserstoff oder Fluor und $R^3$ Wasserstoff bedeuten, oder $R^2$ Wasserstoff und $R^3$ Chlor, Brom, Jod oder Cyano bedeuten. Das Symbol X bedeutet vorzugsweise ein Sauerstoffatom. Die bevorzugte Bedeutungsmöglichkeit von $R^1$ ist Methyl.

Im Rahmen der vorliegenden Erfindung ganz besonders bevorzugte Verbindungen sind t-Butyl-7-chlor-5,6-dihydro-5-methyl-6-oxo-4 H-imidazo[1,5-a][1,4]benzodiazepin-3-carboxylat, t-Butyl-5,6-dihydro-7-jod-5-methyl-6-oxo-4 H-imidazo[1,5-a][1,4]benzodiazepin-3-carboxylat und t-Butyl-7-brom-5,6-dihydro-5-methyl-6-oxo-4 H-imidazo[1,5-a][1,4]benzodiazepin-3-carboxylat.

Weitere bevorzugte, von der allgemeinen Formel I umfaßte Verbindungen sind:

t-Butyl-7-cyano-5,6-dihydro-5-methyl-6-oxo-4 H-imidazo[1,5-a][1,4]benzodiazepin-3-carboxylat,
t-Butyl-5,6-dihydro-5-methyl-6-oxo-4 H-imidazo[1,5-a][1,4]benzodiazepin-3-carboxylat,
t-Butyl-5,6-dihydro-5-methyl-6-thioxo-4 H-imidazo[1,5-a][1,4]benzodiazepin-3-carboxylat und
t-Butyl-8-fluor-5,6-dihydro-5-methyl-6-oxo-4 H-imidazo[1,5-a][1,4]benzodiazepin-3-carboxylat.

Die Imidazodiazepine der allgemeinen Formel I und ihre pharmazeutisch annehmbaren Säureadditionssalze können erfindungsgemäß hergestellt werden, indem man

a) eine Verbindung der allgemeinen Formel

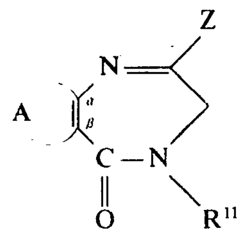

(II)

worin $R^{11}$ niederes Alkyl wie vorher angegeben und Z eine Abgangsgruppe bedeuten, und A obige Bedeutung besitzt, in Gegenwart einer Base mit dem Isocyanessigester der Formel

$$CN—CH_2—COOC(CH_3)_3 \qquad (III)$$

umsetzt, oder

b) in einer Verbindung der allgemeinen Formel

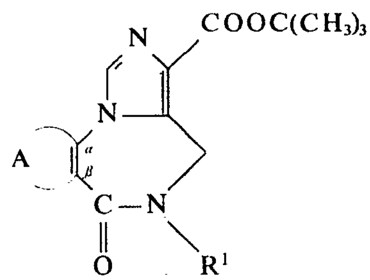

(Ia)

worin $R^1$ und A obige Bedeutung besitzen, die Carbonyl-Gruppe in die Thiocarbonyl-Gruppe überführt, oder

c) eine Carbonsäure der allgemeinen Formel

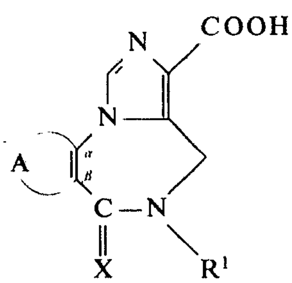

(IV)

worin A, $R^1$ und X obige Bedeutung besitzen, in den entsprechenden t-Butylester überführt, oder

d) eine Verbindung der allgemeinen Formel

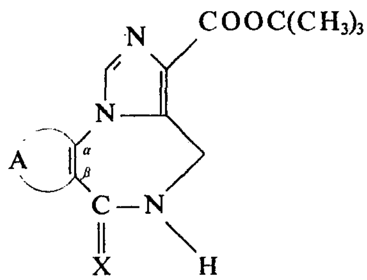

(Ib)

worin A und X obige Bedeutung besitzen, an der sekundären Aminogruppe entsprechend substituiert, oder

e) in einer Verbindung der allgemeinen Formel

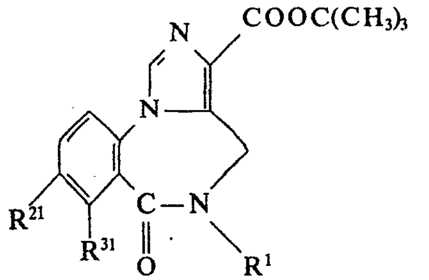

(Ic)

worin einer der Reste $R^{21}$ und $R^{31}$ Halogen und der andere Wasserstoff bedeutet und $R^1$ obige Bedeutung besitzt,
das Halogenatom durch die Cyanogruppe ersetzt, und

f) erwünschtenfalls eine erhaltene Verbindung der allgemeinen Formel I in ein pharmazeutisch annehmbares Säureadditionssalz überführt.

Gemäß Verfahrensvariante a) können Verbindungen der allgemeinen Formel I demnach aus Verbindungen der allgemeinen Formel II und dem Isocyanessigsäureester der Formel III hergestellt werden. Die in Formel II mit Z bezeichnete Abgangsgruppe ist beispielsweise eine leicht abspaltbare Phosphinylgruppe, z. B. eine Gruppe der Formel

$$-\overset{O}{\overset{\|}{O}P}(OR^4)_2 \quad \text{oder} \quad -\overset{O}{\overset{\|}{O}P}(NR^5R^6)_2$$

worin $R^4$ niederes Alkyl und $R^5$ und $R^6$ je niederes Alkyl, Allyl, Phenyl oder substituiertes Phenyl oder zusammen mit dem Stickstoffatom einen unsubstituierten oder substituierten heterocyclischen Ring mit 3 bis 8 Gliedern (wie Morpholin) bedeuten,
ein Halogenatom, eine Alkylthiogruppe, eine Aralkylthiogruppe, eine N-Nitrosoalkylaminogruppe, eine Alkoxygruppe, eine Mercaptogruppe und dergleichen (wenn Z eine Mercaptogruppe bedeutet, so handelt es sich bei der entsprechenden Verbindung der Formel II um die Iminothiol-Form des entsprechenden Thiolactams). Die Umsetzung der Verbindungen der Formeln II und III erfolgt in einem inerten Lösungsmittel, wie Dimethylformamid, Hexamethylphosphorsäuretriamid, Dimethylsulfoxid, Tetrahydrofuran oder irgend einem anderen geeigneten organischen Lösungsmittel und in Gegenwart einer Base, die genügend stark basisch ist, um das Anion des Isocyanessigesters der Formel III zu bilden. Als Basen eignen sich Alkalimetallalkoxide, wie Natriummethoxid oder Kalium-t-butoxid, Alkalimetallhydride, wie Natriumhydrid, Alkalimetallamide, wie Lithiumamid oder Lithiumdiisopropylamid, tertiäre Amine, wie Triäthylamin, und dergleichen. Die Reaktionstemperatur liegt zweckmäßigerweise zwischen etwa −40°C und etwa Raumtemperatur.

Gemäß Verfahrensvariante b) können Verbindungen der allgemeinen Formel Ia in entsprechende Verbindungen der Formel I, worin X ein Schwefelatom bedeutet, übergeführt werden, und zwar durch Behandeln mit einem Schwefelungsmittel, was in an sich bekannter Weise erfolgen kann. Beispielsweise kann man als Schwefelungsmittel Phosphorpentasulfid verwenden, wobei dieses vorzugsweise im Überschuß eingesetzt wird und die Reaktion vorteilhafterweise in einem inerten organischen Lösungsmittel, wie Dioxan, Methylenchlorid oder dergleichen, in Gegenwart von Triäthylamin bei einer Temperatur von etwa 50°C bis zur Rückflußtemperatur des Reaktionsgemisches erfolgt. Als Schwefelungsmittel eignen sich indessen auch Verbindungen wie 2,4-Bis(p-methoxyphenyl)-1,3,2,4-dithiaphosphetan-2,4-disulfid; derartige Schwefelungsmittel werden ungefähr in der berechneten Menge eingesetzt, und die Reaktion erfolgt in Gegenwart eines inerten Lösungsmittels, wie Toluol, Xylol, zweckmäßigerweise bei Rückflußtemperatur des Reaktionsgemisches oder in Hexamethyl-phosphorsäuretriamid zwischen etwa 60° und 110°C.

Gemäß Verfahrensvariante c) können Verbindungen der allgemeinen Formel I hergestellt werden, indem man Carbonsäuren der allgemeinen Formel IV in die entsprechenden t-Butylester überführt. Diese Veresterung kann nach an sich bekannten und jedem Fachmann geläufigen Methoden durchgeführt werden. Beispielsweise kann man eine Carbonsäure der allgemeinen Formel IV mit einem geeigneten Reagens, z. B. Thionylchlorid, Phosphoroxychlorid, Oxalylchlorid oder dergleichen, in das entsprechende Carbonsäurechlorid überführen, und dieses in Gegenwart eines säurebindenden Mittels mit t-Butanol umsetzen. Als säurebindende Mittel eignen sich in erster Linie tertiäre Amine, wie Triäthylamin, Pyridin, Chinuclidin oder dergleichen. Unter Umständen kann die Anwesenheit einer katalytischen Menge an 4-Dimethylaminopyridin oder eines ähnlich reaktiven Amins vorteilhaft sein. Diese Veresterung kann in zwei getrennten Stufen, d. h. Bildung des reaktiven Carbonsäurederivates und Umsetzen derselben mit t-Butanol, oder in einem sogenannten Eintopfverfahren, was bevorzugt wird, durchgeführt werden. Die Reaktionstemperatur liegt zweckmäßigerweise in einem Bereich von etwa −10°C bis Siedetemperatur des Reaktionsgemisches.

Gemäß Verfahrensvariante d) können Verbindungen der allgemeinen Formel I dadurch hergestellt

werden, daß man Verbindungen der Formel Ib an der sekundären Aminogruppe in 5-Stellung entsprechend substituiert. Diese Substitution erfolgt nach an sich bekannten Methoden unter Verwendung eines den gewünschten niederen Alkylrest abgebenden Mittels, beispielsweise entsprechende organische Sulfonsäure-Alkylester (z. B. p-Toluolsulfonsäure-methylester), entsprechende Dialkylsulfate, wie Dimethylsulfat und Diäthylsulfat, entsprechende Alkylhalogenide, wie Methyljodid, Äthyljodid oder Äthylbromid, und dergleichen. Die Verbindung der allgemeinen Formel Ib wird dabei zweckmäßigerweise in Form eines Alkalimetall-Salzes eingesetzt; man erreicht dies zweckmäßigerweise dadurch, daß man die Reaktion in Gegenwart einer starken Base ablaufen läßt oder die Verbindung der Formel Ib vor der Reaktion mit dem Alkylierungsmittel in ein Alkalimetallsalz überführt. Geeignete Basen sind Alkalimetall-alkoxide, wie Natriummethoxid oder Kalium-t-butoxid, Alkalimetall-hydride, wie Natriumhydrid, Alkalimetall-amide, wie Lithiumamid oder Lithiumdiisopropylamid, und dergleichen. Die Reaktion wird zweckmäßigerweise in Gegenwart eines inerten organischen Lösungsmittels durchgeführt. Hierfür eignen sich z. B. Dimethylformamid, Dimethylsulfoxid, Essigester, niedere Alkanole und dergleichen; viele andere Lösungsmittel und auch Lösungsmittelgemische sind ebenfalls geeignet und ihre Auswahl bietet dem Fachmann keinerlei Schwierigkeiten. Die Reaktionstemperatur ist in ziemlich weiten Grenzen variierbar und wird in der Regel zwischen etwa Raumtemperatur und etwa der Siedetemperatur des Reaktionsgemisches liegen.

Gemäß Verfahrensvariante e) können Verbindungen der allgemeinen Formel I, worin $R^2$ oder $R^3$ Cyano bedeutet, hergestellt werden, indem man in Verbindungen der allgemeinen Formel Ic das Halogenatom durch die Cyanogruppe ersetzt. Dabei setzt man vorzugsweise eine entsprechende Brom- oder Jodverbindung der allgemeinen Formel Ic als Ausgangsstoff ein. Die Reaktion kann beispielsweise so durchgeführt werden, daß man die Verbindung der Formel Ic in einem inerten organischen Lösungsmittel mit Kupfer(I)cyanid umsetzt. Geeignete Lösungsmittel sind beispielsweise Dimethylformamid und dergleichen. Die Reaktionstemperatur liegt zweckmäßigerweise in einem Bereich von etwa Raumtemperatur bis Siedetemperatur der Reaktionsmischung.

Gemäß Verfahrensvariante f) können Verbindungen der allgemeinen Formel I erfindungsgemäß in pharmazeutisch annehmbare Säureadditionssalze übergeführt werden. Die Herstellung von solchen pharmazeutisch annehmbaren Säureadditionssalzen erfolgt nach allgemein üblichen Methoden. Es kommen sowohl Salze mit anorganischen als auch Salze mit organischen Säuren in Betracht, beispielsweise Hydrochloride, Hydrobromide, Sulfate, Methansulfonate, p-Toluolsulfonate, Oxalate und dergleichen.

Die als Ausgangsstoffe verwendeten Verbindungen der allgemeinen Formel II können ausgehend von Verbindungen der Formel

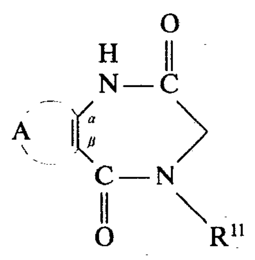

(V)

worin A und $R^{11}$ obige Bedeutung besitzen,
hergestellt werden, und zwar nach Methoden, welche an sich bekannt sind, vgl. z. B. die Belgischen Patentschriften No. 802 233, 833 249 und 865 653, die Amerikanische Patentschrift No. 3 681 341 und J. Org. Chemistry 29, 231 (1964).

Verschiedene der weiter unten folgenden Beispiele enthalten detaillierte Angaben betreffend die Herstellung von Verbindungen der allgemeinen Formel II aus Verbindungen der allgemeinen Formel V.

Die Verbindungen der Formel V ihrerseits sind bekannt oder nach an sich bekannten Methoden leicht herstellbar, beispielsweise durch Umsetzen eines entsprechenden Carbonsäureanhydrids der allgemeinen Formel

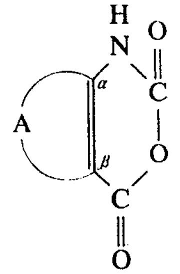

(VI)

worin A obige Bedeutung besitzt,
mit einer Aminosäure der allgemeinen Formel

$$R^{11}—NH—CH_2—COOH \qquad (VII)$$

worin $R^{11}$ obige Bedeutung besitzt.

Die Verbindungen der Formel V können aber auch ausgehend von Verbindungen der allgemeinen Formel

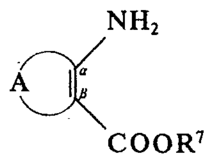

(VIII)

worin $R^7$ niederes Alkyl bedeutet wie vorher angegeben und A obige Bedeutung besitzt, hergestellt werden, beispielsweise durch Behandeln einer solchen Verbindung mit einem reaktiven Derivat einer $\alpha$-Halogenessigsäure, z. B. $\alpha$-Chloressigsäurechlorid, und Umsetzen des erhaltenen Zwischenproduktes mit einem niederen Alkylamin, wie Methylamin, Äthylamin und dergleichen. Man erhält somit Verbindungen der allgemeinen Formel

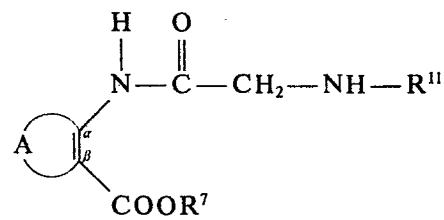

(IX)

worin A, $R^{11}$ und $R^7$ obige Bedeutung besitzen.

Durch Cyclisieren von Verbindungen der allgemeinen Formel IX gelangt man zu Verbindungen der allgemeinen Formel V. Diese Cyclisierung erfolgt beispielsweise durch kurzzeitiges Erhitzen einer entsprechenden Verbindung der allgemeinen Formel IX auf Temperaturen von etwa 100° bis etwa 300°C.

Es ist auch möglich eine Verbindung der Formel VIII mit einem reaktiven Derivat einer Carbonsäure der allgemeinen Formel

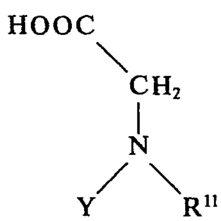

(X)

worin Y eine Schutzgruppe bedeutet und $R^{11}$ obige Bedeutung besitzt, beispielsweise einem Carbonsäurechlorid oder dergleichen, umzusetzen. Nach Entfernen der mit Y bezeichneten Schutzgruppe aus einer so erhaltenen Verbindung der allgemeinen Formel

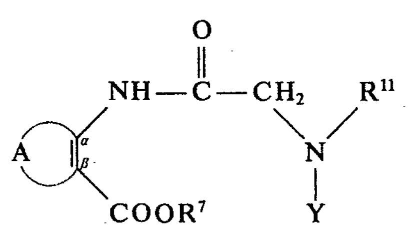

(XI)

worin A, $R^{11}$, $R^7$ und Y obige Bedeutung besitzen,
und Cyclisieren, in Analogie zur Herstellung von Verbindungen der Formel V aus solchen der Formel IX, erhält man eine Verbindung der allgemeinen Formel V.

Die als Ausgangsstoffe verwendeten Verbindungen der allgemeinen Formel IV können durch Hydrolyse der Estergruppe in Verbindungen der allgemeinen Formel

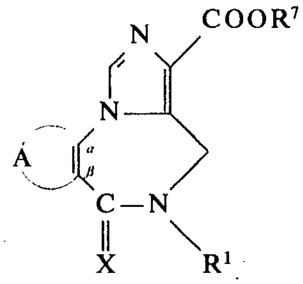

(XII)

worin A, $R^1$, $R^7$ und X obige Bedeutung besitzen, nach an sich bekannten und jedem Fachmann geläufigen Methoden leicht hergestellt werden.

Verbindungen der Formel XII, worin $R^1$ niederes Alkyl bedeutet, können hergestellt werden, indem man eine Verbindung der Formel II in Gegenwart einer Base mit einem Isocyanessigester der allgemeinen Formel

$$CN—CH_2—COOR^7 \qquad \text{(XIII)}$$

worin $R^7$ obige Bedeutung besitzt, umsetzt [in Analogie zu Verfahrensvariante a)], und erwünschtenfalls im erhaltenen Produkt die Carbonyl-Gruppe in die Thiocarbonyl-Gruppe überführt [in Analogie zu Verfahrensvariante b)].

Verbindungen der allgemeinen Formel XII, worin $R^1$ Wasserstoff bedeutet, können hergestellt werden, indem man eine Verbindung der allgemeinen Formel

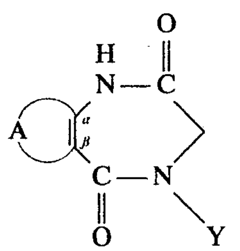

(XIV)

worin A und Y obige Bedeutung besitzen, in Analogie zu Verfahrensvariante a) mit einem Isocyanessigester der allgemeinen Formel XIII umsetzt und im erhaltenen Produkt der allgemeinen Formel

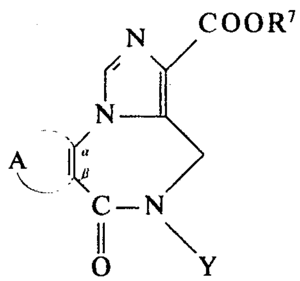

(XV)

worin A, $R^7$ und Y obige Bedeutung besitzen, die mit Y bezeichnete Schutzgruppe abspaltet und vorgängig oder anschließend die Carbonyl-Gruppe in die Thiocarbonyl-Gruppe überführt.

Es kommen dabei nur Schutzgruppen in Frage, die unter milden sauren Bedingungen abgespalten werden können, beispielsweise mit verdünnten wäßrigen Mineralsäuren, wie verdünnte Salzsäure oder verdünnte Schwefelsäure, Trifluoressigsäure oder dergleichen, gegebenenfalls unter Zusatz eines Lösungsvermittlers, wie Tetrahydrofuran, Dioxan, Essigsäure, N,N-Dimethylformamid oder dergleichen. Zweckmäßigerweise arbeitet man bei Temperaturen von etwa Raumtemperatur bis Siedetemperatur der Reaktionsmischung, wobei letzteres bevorzugt wird. Eine besonders geeignete Schutzgruppe ist die 2,4-Dimethoxybenzyl-Gruppe, die zweckmäßigerweise mit Trifluoressigsäure abgespalten wird, vorzugsweise bei der Siedetemperatur des Reaktionsgemisches.

Bedeutet $R^7$ in einer Verbindung der allgemeinen Formel XV t-Butyl, so kann unter den Bedingungen der Schutzgruppenabspaltung gleichzeitig die Estergruppe hydrolysiert werden; man erhält somit direkt eine Verbindung der allgemeinen Formel IV, worin $R^1$ Wasserstoff bedeutet.

Wie eingangs erwähnt, sind die Verbindungen der allgemeinen Formel I neu und besitzen äußerst wertvolle pharmakodynamische Eigenschaften. Sie weisen nur eine geringe Toxizität auf, und es hat

sich gezeigt, daß sie eine ausgeprägte Affinität zu den zentralen Benzodiazepin-Rezeptoren haben und die zentral dämpfenden, muskelrelaxierenden, ataktischen, blutdrucksenkenden und atemdepressiven Eigenschaften von tranquilisierend wirksamen 1,4-Benzodiazepinen zu antagonisieren vermögen.

Die Affinität von Verbindungen der allgemeinen Formel I zu den zentralen Benzodiazepin-Rezeptoren wurde nach der in Life Science 20, 2101–2110 (1977) und Science 198, 849–851 (1977) beschriebenen Methode festgestellt. Nach dieser Methode ermittelt man die Hemmung der Bindung von tritiiertem Diazepam an die spezifischen Benzodiazepin-Rezeptoren im cerebralen Cortex durch die jeweiligen Testsubstanzen. Man bezeichnet als $IC_{50}$ (»50% inhibiting concentration«) diejenige Konzentration der jeweiligen Testsubstanz, welche eine 50proz. Hemmung der spezifischen Bindung des tritiierten Diazepams an die spezifischen Benzodiazepin-Rezeptoren im cerebralen Cortex bewirkt.

Eine der typischen Eigenschaften von tranquilisierend wirksamen 1,4-Benzodiazepinen in Versuchstieren ist ihre ausgeprägte anticonvulsive Wirkung, welche beispielsweise im bekannten und allgemein anerkannten Pentetrazol-Test nachgewiesen werden kann. Diese Eigenschaft wurde verwendet, um den nachstehend beschriebenen Test auszuarbeiten, der es erlaubt, Verbindungen zu ermitteln, die die zentralen Eigenschaften von transquilisierend wirksamen 1,4-Benzodiazepinen zu antagonisieren vermögen.

In diesem Test verabreicht man Mäusen eine Stunde vor dem Pentetrazol (120 mg/kg, i. p.) 5 mg/kg (i. p.) Diazepam (d. h. eine supramaximale Dosis, die im Pentetrazol-Test an mehr als 900 Mäusen alle Versuchstiere vor krampfartigen Anfällen schützte) und 15 Minuten vor dem Pentetrazol die zu testende Verbindung p. o. Die antagonistische Wirksamkeit der untersuchten Verbindungen, d. h. ihr Vermögen, die Wirkung des Diazepams im Pentetrazol-Test aufzuheben, wird ermittelt, indem man die Mäuse auszählt, die in diesem Test krampfartige Anfälle erleiden.

In der nachstehenden Tabelle werden die Resultate dargestellt, welche mit repräsentativen Vertretern der durch die allgemeine Formel I definierten Verbindungsklasse in dem vorstehend geschilderten Versuch erhalten worden sind. Angegeben wird für jede der darin figurierenden Verbindung der $ED_{50}$-Wert. Als $ED_{50}$ bezeichnet man die Menge der jeweiligen Testverbindung in mg/kg (p. o.), die bei 50% der Tiere den Diazepam-Effekt in obigem Versuch aufhebt. Außerdem enthält die Tabelle die oben definierten $IC_{50}$-Werte für alle darin angegebenen Testverbindungen sowie Angaben über die akute Toxizität einiger dieser Verbindungen ($DL_{50}$ in mg/kg bei einmaliger oraler Verabreichung an Mäusen).

Tabelle

| Verbindung der Formel I, worin | | | | | $IC_{50}$ in nM/l | $ED_{50}$ in mg/kg p. o. | $DL_{50}$ in mg/kg p. o. |
|---|---|---|---|---|---|---|---|
| A | $R^2$ | $R^3$ | $R^1$ | X | | | |
| (a) | H | Cl | $-CH_3$ | O | 3,0 | 0,2 | 312 |
| (a) | H | CN | $-CH_3$ | O | 10,0 | 1,22 | |
| (a) | H | H | $-CH_3$ | O | 3,3 | 4,7 | 625 |
| (a) | F | H | $-CH_3$ | O | 1,4 | 8,4 | |
| (a) | H | H | $-CH_3$ | S | 8,9 | 7,1 | |
| (a) | H | J | $-CH_3$ | O | 2,6 | 0,14 | |
| (a) | H | Br | $-CH_3$ | O | 2,3 | 0,13 | |

Wie bereits erwähnt, antagonisieren die Verbindungen der Formel I die zentral dämpfenden, muskelrelaxierenden, ataktischen, blutdrucksenkenden und atemdepressiven Eigenschaften von tranquilisierend wirksamen 1,4-Benzodiazepinen. Letztere stehen in der Therapie in vielfältigem Gebrauch und werden oft in hohen Dosierungen angewandt, so daß die oben erwähnten Wirkungen auch als Nebenwirkungen stark hervortreten können. Die Verbindungen der Formel I können bei Intoxikationen, bei welchen übermäßige Einnahme von tranquilisierend wirksamen 1,4-Benzodiazepinen beteiligt ist, als Antidot verwendet werden. Sie eignen sich auch zur Abkürzung einer durch tranquilisierend wirksame 1,4-Benzodiazepine eingeleiteten Anästhesis in der Chirurgie und in der Geburtshilfe. Bei Neugeborenen kann eine eventuelle Atemdepression, die auf die Gabe von tranquilisierend wirksamen 1,4-Benzodiazepinen an die Mutter zurückgeht, aufgehoben werden. Die Verbindungen der Formel I können auch dazu verwendet werden, bei 1,4-Benzodiazepinen, die in anderen Indikationsgebieten eingesetzt werden, die in einem solchen Fall unerwünschten Wirkungen auf das zentrale Nervensystem zu unterdrücken. Beispiele derartiger, in anderen Indikationsgebieten einsetzbarer 1,4-Benzodiazepine sind die

in den Britischen Patentschriften No. 1 444 529 und 1 474 305 beschriebenen schistosomizid wirksamen 1,4-Benzodiazepine, wie das (+)-5-(o-Chlorphenyl)-1,3-dihydro-3-methyl-7-nitro-2 H-1,4-benzodiazepin-2-on.

Die nachstehenden Experimente zeigen, daß ein repräsentativer Vertreter der von der allgemeinen Formel I umfassten Stoffklasse, welcher die starken, jedoch unerwünschten zentralen Wirkungen des hochwirksamen Schistosomizids (+)-5-(o-Chlorphenyl)-1,3-dihydro-3-methyl-7-nitro-2 H-1,4-benzodiazepin-2-on auf das zentrale Nervensystem unterdrückt, dessen schistosomizide Wirkung in keiner Weise beeinträchtigt.

Mäuse und Goldhamster werden subcutan mit 60 Cercarien von Schistosoma mansoni infiziert. Cirka 42 Tage nach der Infektion werden sie mit einer einmaligen Dosis der zu prüfenden Präparate oral behandelt. Pro Präparat und Dosierung werden 5 Tiere eingesetzt. Als Kontrolle dienen 10 unbehandelte Tiere. Die Tiere werden 2 (Hamster) bzw. 3 (Mäuse) Wochen nach der Behandlung getötet und seziert. Wurmpaare in Mesenterialvenen, Pfortader und Leber werden herauspräpariert, gezählt und der Zustand der Würmer (lebend-tot) wird registriert. Eine schistosomizide Wirkung eines Präparats zeigt sich im Auftreten toter Würmer in den Gefäßen der Leber. In unbehandelten Kontrolltieren finden sich nie tote Würmer. Zur Auswertung errechnet man den prozentualen Anteil toter Wurmpaare in den Gefäßen der Leber bei infizierten, behandelten Tieren.

Zur Prüfung der in vitro Wirkung von Präparaten werden Wurmpaare von Schistosoma mansoni aus Mäusen isoliert und in einem Nährmedium bei 37°C inkubiert. Präparate werden als Lösung oder als Suspension beigegeben. Die Beweglichkeit der Würmer wird während der Versuchsdauer von 120 Stunden unter dem Mikroskop beobachtet und registriert. Eine schistosomizide Wirkung eines Präparats zeigt sich am mehr oder weniger raschen Verlust der Motilität der Würmer. Kontrollwürmer in Nährmedium ohne Präparatzusatz behalten ihre normale Beweglichkeit während der ganzen Versuchsdauer von 120 Stunden.

Die folgende repräsentative Verbindung der Formel I wurde in den oben beschriebenen Versuchen auf mögliche Beeinträchtigung der schistosomiziden Wirkung von (+)-5-(o-Chlorphenyl)-1,3-dihydro-3-methyl-7-nitro-2 H-1,4-benzodiazepin-2-on (Verbindung S) geprüft:

t-Butyl-7-chlor-5,6-dihydro-5-methyl-6-oxo-4 H-imidazo[1,5-a][1,4]benzodiazepin-3-carboxylat (Verbindung A).

Wie aus den folgenden Zusammenstellungen über die Versuchsergebnisse am Versuchstier und im in vitro Versuch hervorgeht, wurde die schistosomizide Wirkung von (+)-5-(o-Chlorphenyl)-1,3-dihydro-3-methyl-7-nitro-2 H-1,4-benzodiazepin-2-on (Verbindung S) von der Verbindung A nicht nachteilig beeinflußt.

Versuchsergebnisse an Maus und Hamster

| Wirtsspezies | Dosierung in mg/kg p.o. | | Dosierungs-verhältnis | schistosomizide Wirkung in % |
|---|---|---|---|---|
| | Verbindung S | Verbindung A | | |
| Maus | 75 | – | – | 100 |
| | – | 300 | – | 0 |
| | 75 | 25 | 3 : 1 | 100 |
| | 75 | 75 | 1 : 1 | 100 |
| | 75 | 225 | 1 : 3 | 93 |
| | – | – | – | 0 |
| Hamster | 75 | – | – | 100 |
| | – | 225 | – | 0 |
| | 75 | 25 | 3 : 1 | 100 |
| | 75 | 75 | 1 : 1 | 100 |
| | 75 | 225 | 1 : 3 | 85 |
| | – | – | – | 0 |

Ergebnisse der in vitro Experimente

| Präparatkonzentration in µg/ml | | Konzentrations-Verhältnis | Wirkung*) |
|---|---|---|---|
| Verbindung S | Verbindung A | | |
| 25 | – | – | a |
| – | 100 | – | b |
| – | 25 | – | b |
| 25 | 100 | 1 : 4 | a |
| 25 | 25 | 1 : 1 | a |
| – | – | – | b |

*) Wirkung:
a = Wurmpaare innert 15 Minuten bewegungslos;
b = Wurmpaare während der Versuchsdauer von 120 Stunden normal beweglich.

Die Verbindungen der allgemeinen Formel I und ihre pharmazeutisch annehmbaren Säureadditionssalze können als Heilmittel, z. B. in Form pharmazeutischer Präparate, Verwendung finden. Die pharmazeutischen Präparate können oral, z. B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z. B. in Form von Suppositorien, oder parenteral, z. B. in Form von Injektionslösungen, erfolgen.

Zur Herstellung von Tabletten, Lacktabletten, Dragées und Hartgelatinekapseln können die Verfahrensprodukte mit pharmazeutisch inerten, anorganischen oder organischen Trägern verarbeitet werden. Als solche Träger kann man für Tabletten, Dragées und Hartgelatinekapseln z. B. Lactose, Maisstärke oder Derivate davon, Talk, Stearinsäure oder deren Salz etc. verwenden. Für Weichgelatinekapseln eignen sich als Träger z. B. pflanzliche Öle, Wachse, Fette, halbfeste und flüssige Polyole etc. Zur Herstellung von Lösungen und Sirupen eignen sich als Träger z. B. Wasser, Polyole, Saccharose, Invertzucker, Glukose und dergleichen. Für Injektionslösungen eignen sich als Träger z. B. Wasser, Alkohole, Polyole, Glyzerin, pflanzliche Öle etc. Für Suppositorien eignen sich als Träger z. B. natürliche oder gehärtete Öle, Wachse, Fette, halbflüssige oder flüssige Polyole und dergleichen.

Die pharmazeutischen Präparate können daneben noch Konservierungsmittel, Lösungsvermittler, Stabilisierungsmittel, Netzmittel, Emulgiermittel, Süßmittel, Färbemittel, Aromatisierungsmittel, Salze zur Veränderung des osmotischen Druckes, Puffer, Überzugsmittel oder Antioxidantien enthalten. Sie können auch noch andere therapeutisch wertvolle Stoffe enthalten.

Wie eingangs erwähnt, kann man Verbindungen der allgemeinen Formel I und pharmazeutisch annehmbare Säureadditionssalze davon erfindungsgemäß bei der Bekämpfung bzw. Verhütung von Krankheiten verwenden, insbesondere bei der Antagonisierung der zentral dämpfenden, muskelrelaxierenden, ataktischen, blutdrucksenkenden und atemdepressiven Eigenschaften von tranquilisierend wirksamen 1,4-Benzodiazepinen. Insbesondere kann man Verbindungen der allgemeinen Formel I in Kombination mit den weiter oben erwähnten schistosomizid wirksamen Verbindungen, z. B. in Kombination mit (+)-5-(o-Chlorphenyl)-1,3-dihydro-3-methyl-7-nitro-2H-1,4-benzodiazepin-2-on, bei der Bekämpfung der Schistosomiasis verwenden. Dabei können die Verbindungen der Formel I oder deren pharmazeutisch annehmbare Säureadditionssalze vor, gleichzeitig mit oder nach der Verabreichung bzw. Einnahme von tranquilisierend wirksamen 1,4-Benzodiazepinen verabreicht werden. Wird die Verbindung der Formel I oder ein pharmazeutisch annehmbares Säureadditionssalz davon gleichzeitig mit dem tranquilisierend wirksamen 1,4-Benzodiazepin verabreicht, so kann dies durch Verabreichung als ad-hoc-Kombination oder in Form einer pharmazeutischen Kombination erfolgen, welche eine Verbindung der allgemeinen Formel I oder ein pharmazeutisch annehmbares Säureadditionssalz davon und ein tranquilisierend wirksames 1,4-Benzodiazepin-Derivat enthält; derartige pharmazeutische Kombinationen sind ebenfalls Gegenstand der vorliegenden Erfindung. Die Dosierung der Verbindungen der Formel I und ihrer pharmazeutisch annehmbaren Säureadditionssalze kann innerhalb weiter Grenzen variieren und ist natürlich in jedem einzelnen Fall den individuellen Gegebenheiten anzupassen. Im allgemeinen dürfte eine Tagesdosis von etwa 0,2 bis etwa 500 mg angemessen sein.

Wie eingangs erwähnt, sind Arzneimittel, enthaltend eine Verbindung der allgemeinen Formel I oder ein pharmazeutisch annehmbares Säureadditionssalz davon, ebenfalls Gegenstand der vorliegenden

Erfindung, weiterhin auch ein Verfahren zur Herstellung solcher Arzneimittel, welches dadurch gekennzeichnet ist, daß man eine oder mehrere Verbindungen der allgemeinen Formel I oder pharmazeutisch annehmbare Säureadditionssalze davon und gegebenenfalls einen oder mehrere andere therapeutisch wertvolle Stoffe in eine galenische Darreichungsform bringt; in diesem Zusammenhang sei nochmals auf die weiter oben erwähnten pharmazeutischen Kombinationen hingewiesen, welche ebenfalls Gegenstand der vorliegenden Erfindung sind. Im speziellen sind pharmazeutische Kombinationen, enthaltend eine Verbindung der allgemeinen Formel I und eine der weiter oben erwähnten schistosomizid wirksamen Verbindungen, insbesondere das (+)-5-(o-Chlorphenyl)-1,3-dihydro-3-methyl-7-nitro-2H-1,4-benzodiazepin-2-on, Gegenstand der vorliegenden Erfindung; derartige Kombinationen eignen sich zur Bekämpfung von Schistosomiasis.

In den nachfolgenden Beispielen, welche die vorliegende Erfindung näher erläutern, ihren Umfang jedoch in keiner Weise beschränken sollen, sind sämtliche Temperaturen in Celsiusgraden angegeben.

## Beispiel 1

a) Man rührt 29,1 g (0,14 Mol) 6-Chlor-isatosäureanhydrid mit 13,12 g (0,14 Mol) Sarcosin in 150 ml Dimethylsulfoxid während 1 Stunde bei 110°. Die erhaltene Lösung wird eingeengt und der Rückstand aus Äthanol umkristallisiert. Man erhält 6-Chlor-3,4-dihydro-4-methyl-2H-1,4-benzodiazepin-2,5(1H)-dion vom Schmelzpunkt 237–238°.

b) Eine Suspension von 0,55 g (14,2 mMol) Natriumhydrid (55proz. Öldispersion) in 20 ml trockenem Dimethylformamid wird mit 3,18 g (14,1 mMol) 6-Chlor-3,4-dihydro-4-methyl-2H-1,4-benzodiazepin-2,5(1H)-dion versetzt. Man rührt während 1 Stunde bei Raumtemperatur, kühlt auf –30° ab und versetzt tropfenweise mit 2,05 ml (14,2 mMol) Diäthylchlorphosphat. Das Reaktionsgemisch wird während 20 Minuten bei –20° gerührt.

Inzwischen kühlt man eine Lösung von 1,59 g (14,2 mMol) Kalium-t-butylat in 5 ml trockenem Dimethylformamid in einem Aceton/Trockeneisbad ab und versetzt mit 2,0 g (14,2 mMol) Isocyanessigsäure-t-butylester. Die erhaltene Lösung wird bei –15° dem obigen Reaktionsgemisch zugetropft. Man entfernt das Kühlbad und neutralisiert das Gemisch bei Raumtemperatur mit 1,5 ml Eisessig. Anschließend gießt man auf ca. 150 ml Wasser und extrahiert dreimal mit Chloroform. Die organischen Auszüge werden viermal mit Wasser gewaschen, über Magnesiumsulfat getrocknet und eingedampft. Das Rohprodukt wird unter Eluieren mit Essigester an Kieselgel chromatographiert und anschließend aus Essigester/Äther umkristallisiert. Man erhält t-Butyl-7-chlor-5,6-dihydro-5-methyl-6-oxo-4H-imidazo[1,5-a][1,4]benzodiazepin-3-carboxylat vom Schmelzpunkt 188–190°.

## Beispiel 2

a) Eine Suspension von 8,7 g (0,03 Mol) 6-Jodisatosäureanhydrid, 2,95 g (0,033 Mol) Sarcosin und 4,2 g Kaliumcarbonat in 50 ml Dimethylformamid wird während 30 Minuten auf 50° erhitzt. Nach dem Abkühlen wird mit 400 ml Wasser verdünnt, mit 2 N Salzsäure auf pH 1–2 gestellt und mehrmals mit Chloroform/Isopropanol (4 : 1) extrahiert. Nach Abdampfen des Lösungsmittels und Umkristallisieren des Rückstandes aus Methylenchlorid/Hexan erhält man N-(6-Jodanthraniloyl)-N-methylglycin vom Schmelzpunkt 147–149°.

b) 4,6 g des obigen Materials werden während ca. 10 Minuten auf 160° erhitzt. Nach Entfernen des während der Reaktion gebildeten Wassers im Vakuum wird das Rohprodukt aus Methylenchlorid/Hexan umkristallisiert. Man erhält 3,4-Dihydro-6-jod-4-methyl-2H-1,4-benzodiazepin-2,5(1H)-dion vom Schmelzpunkt 217–220°.

c) Eine Suspension von 0,28 g (6,6 mMol) Natriumhydrid (55proz. Öldispersion) in 10 ml trockenem Dimethylformamid wird mit 1,94 g (6,1 mMol) 3,4-Dihydro-6-jod-4-methyl-2H-1,4-benzodiazepin-2,5(1H)-dion versetzt. Nach 30 Minuten wird auf –35° abgekühlt und tropfenweise mit 1,1 ml (6,6 mMol) Diäthylchlorphosphat versetzt. Anschließend rührt man noch während ca. 15 Minuten bei –35 bis –15°.

Inzwischen kühlt man eine Lösung von 0,72 g (6,6 mMol) Kalium-t-butylat in 4 ml trockenem Dimethylformamid in einem Aceton/Trockeneisbad ab und versetzt mit 0,95 g (6,6 mMol) Isocyanessigsäure-t-butylester. Die erhaltene Lösung wird bei –15 bis –5° dem obigen Reaktionsgemisch zugetropft. Man entfernt das Kühlbad, neutralisiert nach ca. 15 Minuten mit Eisessig, gießt auf 100 ml Wasser und extrahiert dreimal mit Chloroform. Die Chloroformauszüge werden dreimal mit Wasser gewaschen, über Magnesiumsulfat getrocknet und eingedampft. Die Reinigung des Rohproduktes erfolgt durch Chromatographieren an einer Kieselgelsäule und anschließendes Umkristallisieren aus Essigester. Man erhält t-Butyl-5,6-dihydro-7-jod-5-methyl-6-oxo-4H-imidazo[1,5-a][1,4]benzodiazepin-3-carboxylat vom Schmelzpunkt 203–204°.

Beispiel 3

a) Eine Mischung aus 8,7 g (0,03 Mol) 6-Jodisatosäureanhydrid, 3,2 g (0,036 Mol) Sarcosin und 25 ml Dimethylacetamid wird während 1 Stunde unter Rückfluß zum Sieden erhitzt. Nach Abkühlen und Verdünnen mit Wasser wird das Reaktionsgemisch mit Chloroform ausgeschüttelt. Die Chloroformauszüge werden getrocknet und eingedampft. Nach Umkristallisieren des Rohproduktes aus Methylenchlorid/Äther erhält man 3,4-Dihydro-6-jod-4-methyl-2 H-1,4-benzodiazepin-2,5(1 H)-dion vom Schmelzpunkt 214—217°.

b) In Analogie zu den Angaben in Beispiel 2c) erhält man aus dem obigen Material t-Butyl-5,6-dihydro-7-jod-5-methyl-6-oxo-4 H-imidazo[1,5-a][1,4]benzdiazepin-3-carboxylat vom Schmelzpunkt 203—204°.

Beispiel 4

a) Eine Mischung aus 5,7 g (0,018 Mol) 3,4-Dihydro-6-jod-4-methyl-2 H-1,4-benzodiazepin-2,5-(1 H)-dion, 2,4 g (0,026 Mol) Kupfer(I)cyanid und 60 ml Dimethylformamid wird während 45 Minuten auf 50° erhitzt. Nach dem Abkühlen wird das Reaktionsgemisch mit Wasser verdünnt und mehrmals mit Chloroform/Isopropanol (4 : 1) extrahiert. Die vereinigten organischen Auszüge werden über Magnesiumsulfat getrocknet und eingedampft. Das gelbbraune Rohprodukt wird aus Methanol umkristallisiert. Man erhält 2,3,4,5-Tetrahydro-4-methyl-2,5-dioxo-1 H-1,4-benzodiazepin-6-carbonitril vom Schmelzpunkt 253—256°.

b) Eine Suspension von 0,31 g (7,2 mMol) Natriumhydrid (55proz. Öldispersion) in 8 ml trockenem Dimethylformamid wird mit 1,3 g (6,0 mMol) 2,3,4,5-Tetrahydro-4-methyl-2,5-dioxo-1 H-1,4-benzodiazepin-6-carbonitril versetzt. Nach ca. 30 Minuten wird das Reaktionsgemisch auf —35° abgekühlt, tropfenweise mit 1,2 ml (7,2 mMol) Diäthylchlorphosphat versetzt und während 15 Minuten bei —35° bis —15° gerührt.

Inzwischen wird eine Lösung von 0,79 g (7,2 mMol) Kalium-t-butylat in 3 ml trockenem Dimethylformamid in einem Aceton/Trockeneisbad abgekühlt und mit 1,01 g (7,2 mMol) Isocyanessigsäure-t-butylester versetzt. Die erhaltene Lösung wird bei —15 bis —10° dem obigen Reaktionsgemisch zugetropft. Man entfernt das Kühlbad, neutralisiert nach ca. 10 Minuten mit Eisessig, gießt auf 100 ml Wasser und extrahiert dreimal mit Chloroform. Die Chloroformauszüge werden mit Wasser gewaschen, über Magnesiumsulfat getrocknet und eingedampft. Das Rohprodukt wird an Kieselgel unter Eluieren mit Chloroform, das 1,5 % Methanol enthält, chromatographiert und anschließend aus Essigester umkristallisiert. Man erhält t-Butyl-7-cyano-5,6-dihydro-5-methyl-6-oxo-4 H-imidazo[1,5-a][1,4]benzodiazepin-3-carboxylat vom Schmelzpunkt 213—215°.

Beispiel 5

Eine Suspension von 0,55 g (14,2 mMol) Natriumhydrid (55proz. Öldispersion) in 15 ml trockenem Dimethylformamid wird mit 2,69 g (14,16 mMol) 3,4-Dihydro-4-methyl-2 H-1,4-benzodiazepin-2,5(1 H)-dion versetzt und während 1 Stunde bei Raumtemperatur gerührt. Anschließend kühlt man das Reaktionsgemisch auf —30° ab, versetzt tropfenweise mit 2,05 ml (14,2 mMol) Diäthylchlorphosphat und rührt während 20 Minuten bei —20°.
Inzwischen kühlt man eine Lösung von 1,59 g (14,2 mMol) Kalium-t-butylat in 5 ml trockenem Dimethylformamid in einem Aceton/Trockeneisbad ab und versetzt mit 2,0 g (14,2 mMol) Isocyanessigsäure-t-butylester. Die erhaltene Lösung wird bei —15° dem obigen Reaktionsgemisch zugetropft. Man entfernt das Kühlbad, neutralisiert das Gemisch bei Raumtemperatur mit 1,5 ml Eisessig, gießt auf ca. 100 ml Wasser und extrahiert dreimal mit Chloroform. Die Chloroformauszüge werden viermal mit Wasser gewaschen, über Magnesiumsulfat getrocknet und eingedampft. Das Rohprodukt wird durch Chromatographie an einer Kieselgelsäule unter Eluieren mit Essigester und anschließendes Umkristallisieren aus Essigester/Äther gereinigt. Man erhält t-Butyl-5,6-dihydro-5-methyl-6-oxo-4 H-imidazo-[1,5-a][1,4]benzodiazepin-3-carboxylat vom Schmelzpunkt 217—218°.

Beispiel 6

Man versetzt 1 g t-Butyl-5,6-dihydro-5-methyl-6-oxo-4 H-imidazo[1,5-a][1,4]benzodiazepin-3-carboxylat in 50 ml Toluol mit 0,82 g 2,4-Bis(p-methoxyphenyl)-1,3,2,4-dithiadiphosphetan-2,4-disulfid und erhitzt das erhaltene Gemisch während 6 Stunden unter Rückfluß zum Sieden. Man gießt das Reaktionsgemisch auf Wasser und trennt die Toluolphase ab. Nach Trocknen der organischen Phase über Magnesiumsulfat und Eindampfen wird das Rohprodukt an Kieselgel chromatographiert. Man erhält t-Butyl-5,6-dihydro-5-methyl-6-thioxo-4 H-imidazo[1,5-a][1,4]benzodiazepin-3-carboxylat vom Schmelzpunkt 207—208°.

## Beispiel 7

a) Man löst 24 g (132,5 mMol) 5-Fluorisatosäureanhydrid in 140 ml Dimethylsulfoxid und versetzt mit 11,8 g (132,5 mMol) Sarcosin. Die Lösung wird bis zum Ende der Gasentwicklung bei 100° gerührt (Dauer: ca. 1,5 Stunden) und anschließend auf ca. 1,2 l Wasser gegossen. Nach 10minütigem Rühren kristallisiert ein Festkörper aus. Die Kristalle werden abgenutscht, mit 1 l Wasser gewaschen und getrocknet. Man erhält 7-Fluor-3,4-dihydro-4-methyl-2 H-1,4-benzodiazepin-2,5-(1 H)-dion vom Schmelzpunkt 262—263°.

b) Unter einer Argonatmosphäre versetzt man eine Lösung von 10,40 g (50 mMol) 7-Fluor-3,4-dihydro-4-methyl-2 H-1,4-benzodiazepin-2,5(1 H)-dion in 50 ml trockenem Dimethylformamid mit 1,92 g (50 mMol) Natriumhydrid (50proz. Öldispersion) und rührt während 20 Minuten bei Raumtemperatur. Anschließend tropft man bei —20°, 8,62 g (50 mMol) Diäthylchlorphosphat hinzu und rührt noch während 20 Minuten bei —20°.

Inzwischen kühlt man eine Lösung von 5,60 g (50 mMol) Kalium-t-butylat in 15 ml Dimethylformamid in einem Aceton/Trockeneisbad ab und versetzt mit 7,0 g (50 mMol) Isocyanessigsäure-t-butylester. Die erhaltene orange Lösung wird bei —10° bis —20° dem obigen Reaktionsgemisch zugetropft. Man entfernt das Kühlbad. Das Reaktionsgemisch wird während 20 Minuten gerührt, mit 5 ml Eisessig neutralisiert, auf Wasser gegossen und dreimal mit Chloroform ausgeschüttelt. Man wäscht die vereinigten organischen Auszüge fünfmal mit Wasser, trocknet über Magnesiumsulfat und dampft ein. Nach Umkristallisieren aus Essigester erhält man t-Butyl-8-fluor-5,6-dihydro-5-methyl-6-oxo-4 H-imidazo[1,5-a][1,4]benzodiazepin-3-carboxylat vom Schmelzpunkt 228—229°.

## Beispiel 8

a) Unter einer Argonatmosphäre werden 19,0 g (0,10 Mol) 3,4-Dihydro-4-methyl-2 H-1,4-benzodiazepin-2,5(1 H)-dion in 100 ml trockenem Dimethylformamid vorgelegt. Man gibt 15,5 g (0,12 Mol) Kalium-t-butylat zu, wobei die Temperatur von 25° auf 39° steigt. Man kühlt auf Raumtemperatur und tropft zwischen 18 bis 22° 18,2 g (0,105 Mol) Diäthylchlorphosphat zu.
Separat werden 11,2 g (0,10 Mol) Kalium-t-butylat in 30 ml Dimethylformamid gelöst. Diese Lösung wird auf ca. —50° gekühlt und unter Argon mit 11,3 g (0,10 Mol) Isocyanessigsäureäthylester versetzt. Anschließend wird diese Lösung bei 18 bis 23° unter Kühlung zum obigen Reaktionsgemisch getropft. Man rührt während 1 Stunde bei Raumtemperatur, gibt 5 ml Essigsäure zu, gießt dann auf 500 ml Wasser und extrahiert zweimal mit je 200 ml Chloroform. Die vereinigten Chloroformauszüge werden dreimal mit je 300 ml Wasser gewaschen, über Magnesiumsulfat getrocknet und eingedampft. Zum öligen Rückstand gibt man 150 ml Essigester und läßt bei 0° kristallisieren. Man nutscht die ausgeschiedenen Kristalle ab, wäscht sie mit kaltem Essigester und erhält Äthyl-5,6-dihydro-5-methyl-6-oxo-4 H-imidazo[1,5-a][1,4]benzodiazepin-3-carboxylat vom Schmelzpunkt 163—165°. Nach Umkristallisieren aus Essigester zeigt das Produkt einen Schmelzpunkt von 164—165°.

b) Eine Lösung von 14,26 g (0,05 Mol) Äthyl-5,6-dihydro-5-methyl-6-oxo-4 H-imidazo-[1,5-a][1,4]benzodiazepin-3-carboxalat und 2,2 g (0,055 Mol) Natriumhydroxid in 100 ml Äthanol und 20 ml Wasser wird während 45 Minuten unter Rückfluß zum Sieden erhitzt und anschließend mit 55 ml 1 N Salzsäure und 50 ml Wasser versetzt. Nach Abdestillieren des Äthanols wird der entstandene Kristallbrei abgenutscht, mit Wasser gewaschen und getrocknet. Man erhält 5,6-Dihydro-5-methyl-6-oxo-4 H-imidazo[1,5-a][1,4]benzodiazepin-3-carbonsäure vom Schmelzpunkt 287°.

c) Eine Suspension von 2,6 g (10 mMol) 5,6-Dihydro-5-methyl-6-oxo-4 H-imidazo[1,5-a][1,4]benzodiazepin-3-carbonsäure in 20 ml Pyridin wird zuerst mit 25 ml t-Butanol und anschließend bei —5° tropfenweise mit 1,1 ml Phosphoroxychlorid versetzt. Man rührt während 15 Minuten bei —5° und während 48 Stunden bei Raumtemperatur, gießt das erhaltene Reaktionsgemisch auf 250 ml Wasser und extrahiert viermal mit Chloroform. Die Chloroformauszüge werden dreimal mit verdünnter Natronlauge und dreimal mit Wasser gewaschen, über Magnesiumsulfat getrocknet und eingedampft. Nach Umkristallisieren des Rückstandes aus Essigester erhält man t-Butyl-5,6-dihydro-5-methyl-6-oxo-4 H-imidazo[1,5-a][1,4]benzodiazepin-3-carboxylat vom Schmelzpunkt 216—217°.

## Beispiel 9

a) 4,84 g (0,02 Mol) 6-Bromisatosäureanhydrid und 1,78 g (0,02 Mol) Sarcosin werden zusammen fein verrieben und anschließend unter Schutzgas während 6 Minuten auf 260° erhitzt. Nach dem Abkühlen wird das Rohprodukt unter Eluieren mit Chloroform/Methanol (20 : 1) an Kieselgel chromatographiert. Nach Umkristallisieren aus Chloroform/Hexan erhält man 6-Brom-3,4-dihydro-4-methyl-2 H-1,4-benzodiazepin-2,5-(1 H)-dion vom Schmelzpunkt 232—233°.

b) Eine Suspension von 0,98 g (22,4 mMol) Natriumhydrid (55proz. Öldispersion) in 35 ml trockenem Dimethylformamid wird mit 5,26 g (19,5 mMol) 6-Brom-3,4-dihydro-4-methyl-2 H-1,4-benzodiazepin-2,5(1 H)-dion versetzt und während 45 Minuten bei Raumtemperatur gerührt. Anschließend tropft man zum Reaktionsgemisch 3,3 ml (22,4 mMol) Diäthylchlorphosphat und rührt noch während 25 Minuten bei dieser Temperatur.

Inzwischen wird eine Lösung von 2,62 g (23,4 mMol) Kalium-t-butylat in 6 ml trockenem Dimethylformamid in einem Aceton/Trockeneisbad abgekühlt, mit 3,38 g (23,4 mMol) Isocyanessigsäure-t-butylester versetzt und bei −15° dem obigen Reaktionsgemisch zugetropft. Man entfernt das Kühlbad, rührt, bis die Innentemperatur 5° erreicht, neutralisiert mit Eisessig, gießt auf 200 ml Wasser und extrahiert viermal mit Methylenchlorid. Die Methylenchloridauszüge werden dreimal mit Wasser gewaschen, über Magnesiumsulfat getrocknet und eingedampft. Das Rohprodukt wird unter Eluieren mit Essigester/Methylenchlorid (1 : 1) an Kieselgel chromatographiert und anschließend zweimal aus Essigester umkristallisiert. Man erhält t-Butyl-7-brom-5,6-dihydro-5-methyl-6-oxo-4 H-imidazo-[1,5-a][1,4]benzodiazepin-3-carboxylat vom Schmelzpunkt 207—208°.

## Beispiel 10

Eine Mischung aus 5,02 g (22,7 mMol) 6-Chlor-3,4-dihydro-4-methyl-2 H-1,4-benzodiazepin-2,5-(1 H)-dion, 31,5 g (260 mMol) Dimethylanilin, 5,20 g (34 mMol) Phosphoroxychlorid und 50 ml Chloroform (über Aluminiumoxid filtriert) wird während 2 Stunden bei Siedetemperatur gerührt. Man gießt die erhaltene Lösung auf ein vorgekühltes Gemisch aus 18 g Natriumbicarbonat und 100 ml Wasser und rührt während 20 Minuten. Die anorganische Phase wird abgetrennt und dreimal mit Chloroform ausgeschüttelt. Die vereinigten Chloroformauszüge werden über Magnesiumsulfat getrocknet und im Hochvakuum eingedampft. Der kristalline Rückstand (Iminchlorid) wird in 30 ml Dimethylformamid gelöst.

Inzwischen wird eine Lösung von 2,63 g (23,5 mMol) Kalium-t-butylat in 10 ml Dimethylformamid auf −40° abgekühlt, zuerst mit 3,22 g (22,9 mMol) Isocyanessigsäure-t-butylester und anschließend bei −10° bis −20° tropfenweise mit der obigen Lösung des Iminchlorids versetzt. Man entfernt die Kühlung, rührt das Gemisch während 0,5 Stunden, versetzt mit 2,5 ml Eisessig, gießt auf ca. 200 ml Wasser und extrahiert viermal mit je ca. 40 ml Chloroform. Die vereinigten Chloroformauszüge werden dreimal mit Wasser gewaschen, über Magnesiumsulfat getrocknet und eingedampft. Das Rohprodukt wird aus Essigester/Äther umkristallisiert und liefert t-Butyl-7-chlor-5,6-dihydro-5-methyl-6-oxo-4 H-imidazo[1,5-a][1,4]benzodiazepin-3-carboxylat vom Schmelzpunkt 194—196°.

## Beispiel A

Es werden Tabletten folgender Zusammensetzung hergestellt:

| | mg/Tablette |
|---|---|
| t-Butyl-7-chlor-5,6-dihydro-5-methyl-6-oxo-4 H-imidazo[1,5-a][1,4]benzodiazepin-3-carboxylat | 1 |
| Milchzucker | 103 |
| Maisstärke | 25 |
| Mikrokristalline Cellulose | 70 |
| Magnesiumstearat | 1 |
| Total | 200 |

Beispiel B

Es werden Kapseln folgender Zusammensetzung hergestellt:

| | mg/Kapsel |
|---|---|
| t-Butyl-5,6-dihydro-7-jod-5-methyl-6-oxo-4 H-imidazo[1,5-a][1,4]benzo-diazepin-3-carboxylat | 1 |
| Milchzucker | 164 |
| Maisstärke | 30 |
| Talk | 5 |
| Total | 200 |

Der Wirkstoff, Milchzucker und Maisstärke werden zunächst in einem Mischer und dann in einer Zerkleinerungsmaschine vermengt. Man bringt das Gemisch wieder in den Mischer zurück, gibt den Talk zu und vermengt gründlich. Das Gemisch wird maschinell in Hartgelatinekapseln abgefüllt.

Beispiel C

Es werden Injektionslösungen folgender Zusammensetzung hergestellt:

| | pro ml |
|---|---|
| t-Butyl-7-brom-5,6-dihydro-5-methyl-6-oxo-4 H-imidazo[1,5-a][1,4]benzo-diazepin-3-carboxylat | 0,5 mg |
| Propylenglykol | 0,4 ml |
| Äthanol (95proz.) | 0,1 ml |
| Natriumbenzoat | 48,8 mg |
| Benzylalkohol | 0,015 ml |
| Benzoesäure | 1,2 mg |
| Wasser für Injektion q. s. ad | 1,0 |

Zur Herstellung von 10 000 ml Injektionslösung löst man 5 g des Wirkstoffes in 150 ml Benzylalkohol und gibt 4000 ml Propylenglykol und 1000 ml Äthanol zu. Dann löst man 12 g Benzoesäure in dem obigen Gemisch und gibt eine Lösung von 488 g Natriumbenzoat in 300 ml Wasser (für Injektion) zu. Die erhaltene Lösung wird durch Zugabe von Wasser (für Injektion) auf ein Volumen von 10 000 ml gebracht, filtriert und in Ampullen geeigneter Größe abgefüllt; man füllt das Restvolumen der Ampullen mit Stickstoff, schmilzt die Ampullen zu und sterilisiert sie während 30 Minuten im Autoklaven bei 0,7 Atm.

Beispiel D

Es werden Suppositorien folgender Zusammensetzung hergestellt:

| | | g/Supp. |
|---|---|---|
| t-Butyl-7-chlor-5,6-dihydro-5-methyl-6-oxo-4 H-imidazo[1,5-a][1,4]benzodiazepin-3-carboxylat | | 0,001 |
| Cacaobutter (Smp. 36–37°) | | 1,255 |
| Carnauba-Wachs | | 0,044 |
| | Total | 1,3 |

Cacaobutter und Carnauba-Wachs werden in einem Glas- oder Stahlgefäß geschmolzen, gründlich vermengt und auf 45° abgekühlt. Hierauf gibt man den fein pulverisierten Wirkstoff hinzu und rührt, bis er vollständig dispergiert ist. Man gießt die Mischung in Suppositorienformen geeigneter Größe, läßt abkühlen, nimmt dann die Suppositorien aus den Formen und verpackt sie einzeln in Wachspapier oder Metallfolien.

Beispiel E

Es werden Kapseln folgender Zusammensetzung hergestellt:

| | | mg/Kapsel |
|---|---|---|
| (+)-5-(o-Chlorphenyl)-1,3-dihydro-3-methyl-7-nitro-2 H-1,4-benzodiazepin-2-on | | 30,0 |
| t-Butyl-7-chlor-5,6-dihydro-5-methyl-6-oxo-4 H-imidazo[1,5-a][1,4]benzodiazepin-3-carboxylat | | 20,0 |
| Milchzucker krist. | | 100,0 |
| Maisstärke weiß | | 27,5 |
| Talk | | 10,0 |
| Magnesiumstearat | | 2,5 |
| | Total | 190,0 |

Die beiden Wirkstoffe werden mit den Hilfsstoffen gut vermischt und zu je 190,0 mg in Steckkapseln geeigneter Größe abgefüllt.

Beispiel F

Es werden Tabletten folgender Zusammensetzung hergestellt:

| | mg/Tablette |
|---|---|
| (+)-5-(o-Chlorphenyl)-1,3-dihydro-3-methyl-7-nitro-2 H-1,4-benzodiazepin-2-on | 30,0 |
| t-Butyl-7-brom-5,6-dihydro-5-methyl-6-oxo-4 H-imidazo[1,5-a][1,4]benzodiazepin-3-carboxylat | 10,0 |

Fortsetzung

| | mg/Tablette |
|---|---|
| Milchzucker pulvis | 15,0 |
| Maisstärke weiß | 19,5 |
| Povidon K 30 | 3,5 |
| Maisstärke weiß | 10,0 |
| Magnesiumstearat | 2,0 |
| Total | 90,0 |

Die beiden Wirkstoffe, Milchzucker pulvis und die erste Portion Maisstärke weiß werden gemischt und gesiebt. Diese Mischung wird mit einer Lösung von Povidon K 30 in Wasser befeuchtet, geknetet, granuliert, getrocknet und gesiebt. Dem Granulat werden die zweite Portion Maisstärke weiß und Magnesiumstearat zugesetzt. Nach Mischen wird die erhaltene Masse zu 90 mg schweren Tabletten verpreßt.

Beispiel G

Es werden Tabletten folgender Zusammensetzung hergestellt:

| | mg/Tablette |
|---|---|
| (+)-5-(o-Chlorphenyl)-1,3-dihydro-3-methyl-7-nitro-2 H-1,4-benzodiazepin-2-on | 30 |
| t-Butyl-5,6-dihydro-7-jod-5-methyl-6-oxo-4 H-imidazo[1,5-a][1,4]benzodiazepin-3-carboxylat | 30 |
| Milchzucker pulvis | 22 |
| Maisstärke weiß | 22 |
| Povidon K 30 | 6 |
| Maisstärke weiß | 16 |
| Magnesiumstearat | 4 |
| Total | 130 |

Die beiden Wirkstoffe, Milchzucker pulvis und die erste Portion Maisstärke weiß werden gemischt und gesiebt. Diese Mischung wird mit einer Lösung von Povidon K 30 in Wasser befeuchtet, geknetet, granuliert, getrocknet und gesiebt. Dem Granulat werden die zweite Portion Maisstärke weiß und Magnesiumstearat zugesetzt. Nach Mischen wird die erhaltene Masse zu 130 mg schweren Tabletten verpreßt.

**Patentansprüche für die Vertragsstaaten: BE, CH, DE, FR, IT, LI, LU, NL, SE**

1. Imidazodiazepine der allgemeinen Formel

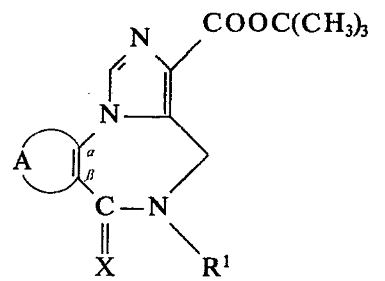

(I)

worin A zusammen mit den beiden mit *α* und *β* bezeichneten Kohlenstoffatomen die Gruppe

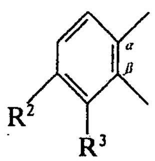

oder

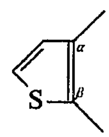

(a) (b)

X ein Sauerstoff- oder Schwefelatom und $R^1$ Wasserstoff oder Alkyl mit 1 bis 7 Kohlenstoffatomen bedeuten, und entweder $R^2$ Wasserstoff, Trifluormethyl, Halogen, Cyano oder Nitro und $R^3$ Wasserstoff bedeuten, oder $R^2$ Wasserstoff und $R^3$ Trifluormethyl, Halogen, Cyano, Nitro oder Alkyl mit 1 bis 7 Kohlenstoffatomen bedeuten,
und pharmazeutisch annehmbare Säureadditionssalze davon.

2. Verbindungen gemäß Anspruch 1, dadurch gekennzeichnet, daß A die in Anspruch 1 definierte Gruppe (a) bedeutet.

3. Verbindungen gemäß Anspruch 2, dadurch gekennzeichnet, daß entweder $R^2$ Wasserstoff oder Fluor und $R^3$ Wasserstoff bedeuten, oder $R^2$ Wasserstoff und $R^3$ Chlor, Brom, Jod oder Cyano bedeuten.

4. Verbindungen gemäß einem der Ansprüche 1 bis 3, dadurch gekennzeichnet, daß X ein Sauerstoffatom bedeutet.

5. Verbindungen gemäß einem der Ansprüche 1 bis 4, dadurch gekennzeichnet, daß $R^1$ Methyl bedeutet.

6. t-Butyl-7-chlor-5,6-dihydro-5-methyl-6-oxo-4H-imidazo[1,5-a][1,4]benzodiazepin-3-carboxylat.

7. t-Butyl-5,6-dihydro-7-jod-5-methyl-6-oxo-4H-imidazo[1,5-a][1,4]benzodiazepin-3-carboxylat.

8. t-Butyl-7-brom-5,6-dihydro-5-methyl-6-oxo-4H-imidazo[1,5-a][1,4]benzodiazepin-3-carboxylat.

9. t-Butyl-7-cyano-5,6-dihydro-5-methyl-6-oxo-4H-imidazo[1,5-a][1,4]benzodiazepin-3-carboxylat, t-Butyl-5,6-dihydro-5-methyl-6-oxo-4H-imidazo[1,5-a][1,4]benzodiazepin-3-carboxylat, t-Butyl-5,6-dihydro-5-methyl-6-thioxo-4H-imidazo[1,5-a][1,4]benzodiazepin-3-carboxylat und t-Butyl-8-fluor-5,6-dihydro-5-methyl-6-oxo-4H-imidazo[1,5-a][1,4]benzodiazepin-3-carboxylat.

10. Verbindungen gemäß einem der Ansprüche 1 bis 9 als pharmazeutische Wirkstoffe.

11. Pharmazeutische Wirkstoffe gemäß Anspruch 10, welche die zentral dämpfenden, muskelrelaxierenden, ataktischen, blutdrucksenkenden und atemdepressiven Eigenschaften von tranquilisierend wirksamen 1,4-Benzodiazepinen antagonisieren.

12. Verfahren zur Herstellung von Verbindungen gemäß einem der Ansprüche 1 bis 9, dadurch gekennzeichnet, daß man

a) eine Verbindung der allgemeinen Formel

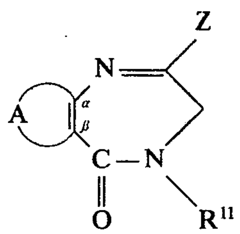

(II)

worin $R^{11}$ Alkyl mit 1 bis 7 Kohlenstoffatomen und Z eine Abgangsgruppe bedeuten, und A die in Anspruch 1 angegebene Bedeutung besitzt,

in Gegenwart einer Base mit dem Isocyanessigester der Formel

$CN-CH_2-COOC(CH_3)_3$ (III)

umsetzt, oder

b) in einer Verbindung der allgemeinen Formel

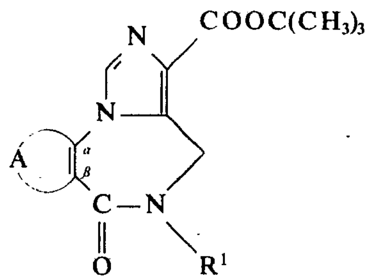

(Ia)

worin A und $R^1$ die in Anspruch 1 angegebene Bedeutung besitzen, die Carbonyl-Gruppe in die Thiocarbonyl-Gruppe überführt, oder

c) eine Carbonsäure der allgemeinen Formel

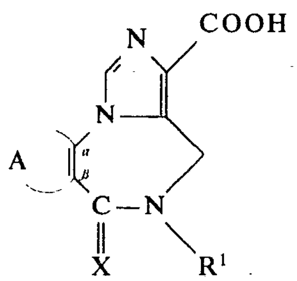

(IV)

worin A, $R^1$ und X die in Anspruch 1 angegebene Bedeutung besitzen, in den entsprechenden t-Butylester überführt, oder

d) eine Verbindung der allgemeinen Formel

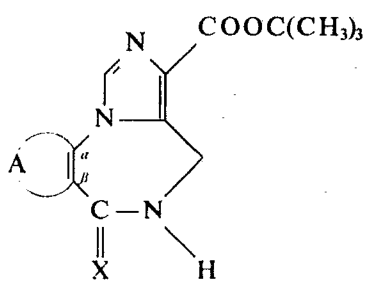

(Ib)

worin A und X die in Anspruch 1 angegebene Bedeutung besitzen, an der sekundären Aminogruppe entsprechend substituiert, oder

e) in einer Verbindung der allgemeinen Formel

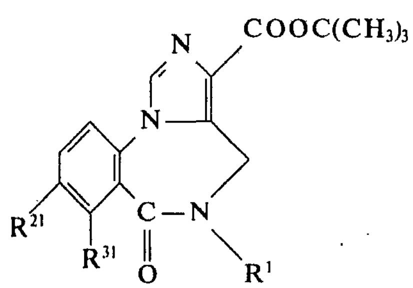

(Ic)

worin einer der Reste $R^{21}$ und $R^{31}$ Halogen und der andere Wasserstoff bedeutet und $R^1$ die in Anspruch 1 angegebene Bedeutung besitzt,

das Halogenatom durch die Cyanogruppe ersetzt, und

f) erwünschtenfalls eine erhaltene Verbindung der allgemeinen Formel I in ein pharmazeutisch annehmbares Säureadditionssalz überführt.

13. Arzneimittel, enthaltend eine Verbindung gemäß einem der Ansprüche 1 bis 9.

14. Arzneimittel gemäß Anspruch 13, welche die zentral dämpfenden, muskelrelaxierenden, ataktischen, blutdrucksenkenden und atemdepressiven Eigenschaften von tranquilisierend wirksamen 1,4-Benzodiazepinen antagonisieren.

15. Schistosomizid wirksames Arzneimittel, enthaltend eine Verbindung gemäß einem der Ansprüche 1 bis 9 und (+)-5-(o-Chlorphenyl)-1,3-dihydro-3-methyl-7-nitro-2 H-1,4-benzodiazepin-2-on.

**Patentansprüche für den Vertragsstaat AT**

1. Verfahren zur Herstellung von Imidazodiazepinen der allgemeinen Formel

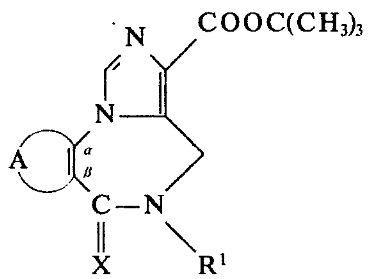

(I)

worin A zusammen mit den beiden mit *α* und *β* bezeichneten Kohlenstoffatomen die Gruppe

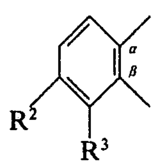

oder

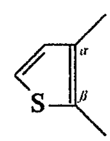

(a) (b)

X ein Sauerstoff- oder Schwefelatom und $R^1$ Wasserstoff oder Alkyl mit 1 bis 7 Kohlenstoffatomen bedeuten, und entweder $R^2$ Wasserstoff, Trifluormethyl, Halogen, Cyano oder Nitro und $R^3$ Wasserstoff bedeuten, oder $R^2$ Wasserstoff und $R^3$ Trifluormethyl, Halogen, Cyano, Nitro oder Alkyl mit 1 bis 7 Kohlenstoffatomen bedeuten,
und pharmazeutisch annehmbaren Säureadditionssalzen davon, dadurch gekennzeichnet, daß man

a) eine Verbindung der allgemeinen Formel

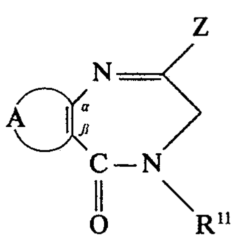

(II)

worin $R^{11}$ Alkyl mit 1 bis 7 Kohlenstoffatomen und Z eine Abgangsgruppe bedeuten, und A obige Bedeutung besitzt,
in Gegenwart einer Base mit dem Isocyanessigester der Formel

$$CN—CH_2—COOC(CH_3)_3 \quad (III)$$

umsetzt, oder

b) in einer Verbindung der allgemeinen Formel

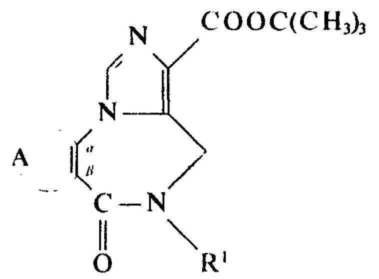

(Ia)

worin A und $R^1$ obige Bedeutung besitzen, die Carbonyl-Gruppe in die Thiocarbonyl-Gruppe überführt, oder

c) eine Carbonsäure der allgemeinen Formel

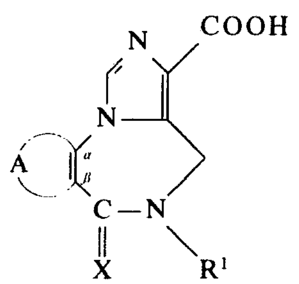

(IV)

worin A, $R^1$ und X obige Bedeutung besitzen, in den entsprechenden t-Butylester überführt, oder

d) eine Verbindung der allgemeinen Formel

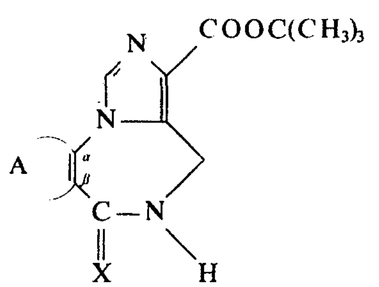

(Ib)

worin A und X obige Bedeutung besitzen, an der sekundären Aminogruppe entsprechend substituiert, oder

e) in einer Verbindung der allgemeinen Formel

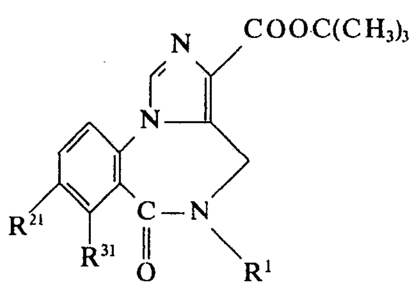

(Ic)

worin einer der Reste $R^{21}$ und $R^{31}$ Halogen und der andere Wasserstoff bedeutet, und $R^1$ obige Bedeutung besitzt, das Halogenatom durch die Cyanogruppe ersetzt, und

f) erwünschtenfalls eine erhaltene Verbindung der allgemeinen Formel I in ein pharmazeutisch annehmbares Säureadditionssalz überführt.

2. Verfahren gemäß Anspruch 1, dadurch gekennzeichnet, daß A die in Anspruch 1 definierte Gruppe (a) bedeutet.

3. Verfahren gemäß Anspruch 2, dadurch gekennzeichnet, daß entweder $R^2$ Wasserstoff oder Fluor und $R^3$ Wasserstoff bedeuten, oder $R^2$ Wasserstoff und $R^3$ Chlor, Brom, Jod oder Cyano bedeuten.

4. Verfahren gemäß einem der Ansprüche 1 bis 3, dadurch gekennzeichnet, daß X ein Sauerstoffatom bedeutet.

5. Verfahren gemäß einem der Ansprüche 1 bis 4, dadurch gekennzeichnet, daß $R^1$ Methyl bedeutet.

6. Verfahren gemäß Anspruch 1, dadurch gekennzeichnet, daß man t-Butyl-7-chlor-5,6-dihydro-5-methyl-6-oxo-4 H-imidazo[1,5-a][1,4]benzodiazepin-3-carboxylat herstellt.

7. Verfahren gemäß Anspruch 1, dadurch gekennzeichnet, daß man t-Butyl-5,6-dihydro-7-jod-5-methyl-6-oxo-4 H-imidazo[1,5-a][1,4]benzodiazepin-3-carboxylat herstellt.

8. Verfahren gemäß Anspruch 1, dadurch gekennzeichnet, daß man t-Butyl-7-brom-5,6-dihydro-5-methyl-6-oxo-4 H-imidazo[1,5-a][1,4]benzodiazepin-3-carboxylat herstellt.

**Claims for the Contracting States: BE, CH, DE, FR, IT, LI, LU, NL, SE**

1. Imidazodiazepines of the general formula

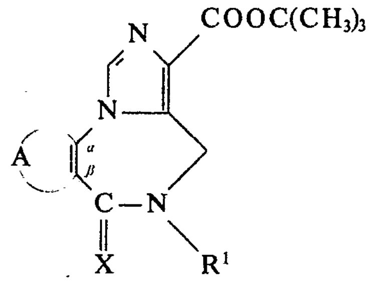

(I)

wherein A together with the two carbon atoms denoted as $\alpha$ and $\beta$ signifies the group

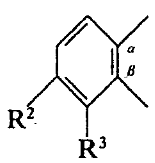

or

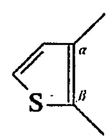

(a) (b)

X signifies an oxygen or sulphur atom and $R^1$ signifies hydrogen or alkyl with 1 to 7 carbon atoms, and either $R^2$ signifies hydrogen, trifluoromethyl, halogen, cyano or nitro and $R^3$ signifies hydrogen, or $R^2$ signifies hydrogen and $R^3$ signifies trifluoromethyl, halogen, cyano, nitro or alkyl with 1 to 7 carbon atoms,
and pharmaceutically acceptable acid addition salts thereof.

2. Compounds in accordance with claim 1, characterized in that A signifies the group (a) defined in claim 1.

3. Compounds in accordance with claim 2, characterized in that either $R^2$ signifies hydrogen or fluorine and $R^3$ signifies hydrogen, or $R^2$ signifies hydrogen and $R^3$ signifies chlorine, bromine, iodine or cyano.

4. Compounds in accordance with any one of claims 1 to 3, characterized in that X signifies an oxygen atom.

5. Compounds in accordance with any one of claims 1 to 4, characterized in that $R^1$ signifies methyl.

6. t-Butyl 7-chloro-5,6-dihydro-5-methyl-6-oxo-4 H-imidazo[1,5-a][1,4]benzodiazepine-3-carboxylate.

7. t-Butyl 5,6-dihydro-7-iodo-5-methyl-6-oxo-4 H-imidazo[1,5-a][1,4]benzodiazepine-3-carboxylate.

8. t-Butyl 7-bromo-5,6-dihydro-5-methyl-6-oxo-4 H-imidazo[1,5-a][1,4]benzodiazepine-3-carboxylate.

9. t-Butyl 7-cyano-5,6-dihydro-5-methyl-6-oxo-4 H-imidazo[1,5-a][1,4]benzodiazepine-3-carboxylate, t-Butyl 5,6-dihydro-5-methyl-6-oxo-4 H-imidazo[1,5-a][1,4]benzodiazepine-3-carboxylate, t-Butyl 5,6-dihydro-5-methyl-6-thioxo-4 H-imidazo[1,5-a][1,4]benzodiazepine-3-carboxylate and t-Butyl 8-fluoro-5,6-dihydro-5-methyl-6-oxo-4 H-imidazo[1,5-a][1,4]benzodiazepine-3-carboxylate.

10. Compounds in accordance with any one of claims 1 to 9 as pharmaceutically active substances.

11. Pharmaceutically active substances in accordance with claim 10, which antagonize the central-depressant, muscle relaxant, ataxic, blood pressure-lowering and respiratory-depressant properties of

1,4-benzodiazepines which have tranquillizing activity.

12. A process for the manufacture of compounds in accordance with any of claims 1 to 9, characterized by

a) reacting a compound of the general formula

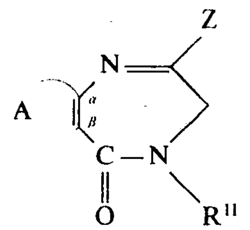

(II)

wherein $R^{11}$ signifies alkyl with 1 to 7 carbon atoms and Z signifies a leaving group, and A has the significance given in claim 1,
in the presence of a base with the isocyanoacetic ester of the formula

$$CN—CH_2—COOC(CH_3)_3$$ (III)

or

b) converting the carbonyl group in a compound of the general formula

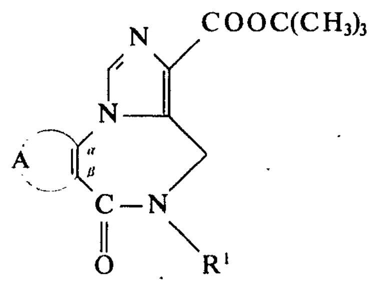

(Ia)

wherein A and $R^1$ have the significance given in claim 1,
into the thiocarbonyl group, or

c) converting a carboxylic acid of the general formula

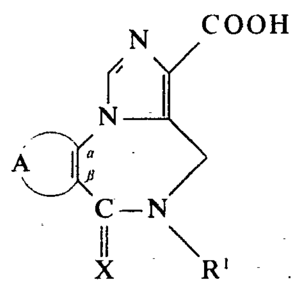

(IV)

wherein A, $R^1$ and X have the significance given in claim 1,
into the corresponding t-butyl ester, or

d) appropriately substituting a compound of the general formula

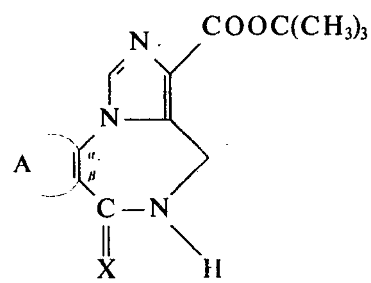

(Ib)

wherein A and X have the significance given in claim 1, at the secondary amino group, or

e) replacing the halogen atom in a compound of the general formula

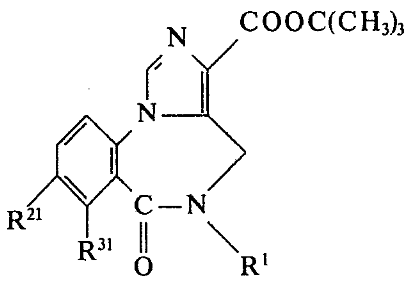

(Ic)

wherein one of the residues $R^{21}$ and $R^{31}$ signifies halogen and the other signifies hydrogen, and $R^1$ has the significance given in claim 1, by the cyano group, and

f) if desired, converting a compound of general formula I obtained into a pharmaceutically acceptable acid addition salt.

13. Medicaments containing a compound in accordance with any one of claims 1 to 9.

14. Medicaments in accordance with claim 13, which antagonize the central-depressant, muscle relaxant, ataxic, blood pressure-lowering and respiratory-depressant properties of 1,4-benzodiazepines which have tranquillizing activity.

15. Schistosomicidally-active medicaments containing a compound in accordance with any one of claims 1 to 9 and (+)-5-(o-chlorophenyl)-1,3-dihydro-3-methyl-7-nitro-2H-1,4-benzodiazepin-2-one.

**Claims for the Contracting State: AT**

1. A process for the manufacture of imidazodiazepines of the general formula

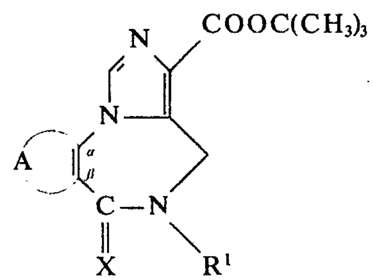

(I)

wherein A together with the two carbon atoms denoted as $\alpha$ and $\beta$ signifies the group

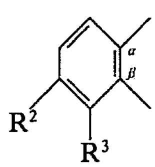

or

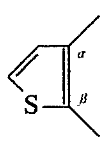

(a) (b)

X signifies an oxygen or sulphur atom and $R^1$ signifies hydrogen or alkyl with 1 to 7 carbon atoms, and either $R^2$ signifies hydrogen, trifluoromethyl, halogen, cyano or nitro and $R^3$ signifies hydrogen, or $R^2$ signifies hydrogen and $R^3$ signifies trifluoromethyl, halogen, cyano, nitro or alkyl with 1 to 7 carbon atoms,

characterized by

a) reacting a compound of the general formula

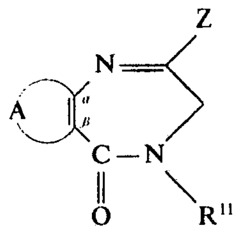

(II)

wherein $R^{11}$ signifies alkyl with 1 to 7 carbon atoms and Z signifies a leaving group, and A has the above significance,
in the presence of a base with the isocyanoacetic ester of the formula

$$CN—CH_2—COOC(CH_3)_3 \quad (III)$$

or

b) converting the carbonyl group in a compound of the general formula

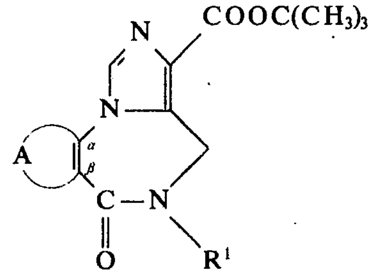

(Ia)

wherein A and $R^1$ have the above significance, into the thiocarbonyl group, or

c) converting a carboxylic acid of the general formula

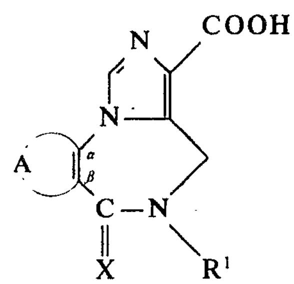

(IV)

wherein A, $R^1$ and X have the above significance,
into the corresponding t-butyl ester, or

d) appropriately substituting a compound of the general formula

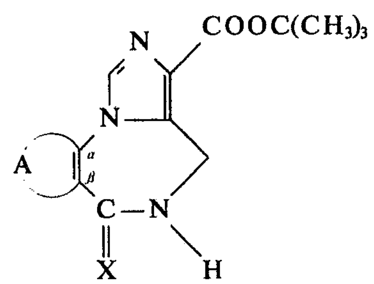

(Ib)

wherein A and X have the above significance, at the secondary amino group, or

e) replacing the halogen atom in a compound of the general formula

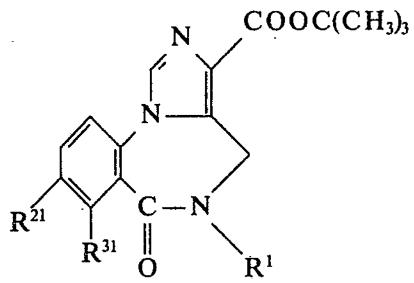

(Ic)

wherein one of the residues $R^{21}$ and $R^{31}$ signifies halogen and the other signifies hydrogen, and $R^1$ has the above significance,
by the cyano group, and

f) if desired, converting a compound of general formula I obtained into a pharmaceutically acceptable acid addition salt.

2. A process in accordance with claim 1, characterized in that A signifies the group (a) defined in claim 1.

3. A process in accordance with claim 2, characterized in that either $R^2$ signifies hydrogen or fluorine and $R^3$ signifies hydrogen, or $R^2$ signifies hydrogen and $R^3$ signifies chlorine, bromine, iodine or cyano.

4. A process in accordance with any one of claims 1 to 3, characterized in that X signifies an oxygen atom.

5. A process in accordance with any one of claims 1 to 4, characterized in that $R^1$ signifies methyl.

6. A process in accordance with claim 1, characterized in that t-butyl 7-chloro-5,6-dihydro-5-methyl-6-oxo-4 H-imidazo[1,5-a][1,4]benzodiazepine-3-carboxylate is manufactured.

7. A process in accordance with claim 1, characterized in that t-butyl 5,6-dihydro-7-iodo-5-methyl-6-oxo-4 H-imidazo[1,5-a][1,4]benzodiazepine-3-carboxylate is manufactured.

8. A process in accordance with claim 1, characterized in that t-butyl 7-bromo-5,6-dihydro-5-methyl-6-oxo-4 H-imidazo[1,5-a][1,4]benzodiazepine-3-carboxylate is manufactured.

**Revendications pour les Etats contractants: BE, CH, DE, FR, IT, LI, NU, NL, SE**

1. Imidazodiazépines de formule générale

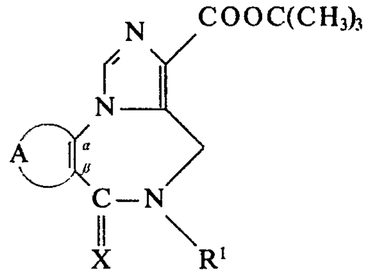

(I)

dans laquelle A forme avec les deux atomes de carbone marqués $\alpha$ et $\beta$ le groupe

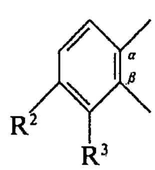

ou

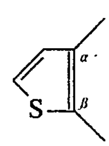

(a) (b)

X représente un atome d'oxygène ou de soufre, et $R^1$ représente l'hydrogène ou un groupe alkyle en C 1—C 7, et ou bien $R^2$ représente l'hydrogène, un groupe trifluorométhyle, un halogène, un groupe cyano ou nitro et $R^3$ représente l'hydrogène, ou bien $R^2$ représente l'hydrogène et $R^3$ représente un groupe trifluorométhyle, un halogène, un groupe cyano, nitro ou alkyle en C 1—C 7,
et leurs sels formés par addition avec des acides acceptables pour l'usage pharmaceutique.

2. Composés selon la rev. 1, caractérisés en ce que A représente le groupe (a) défini dans la rev. 1.

3. Composés selon la rev. 2, caractérisés en ce que, ou bien $R^2$ représente l'hydrogène ou le fluor et $R^3$ l'hydrogène, ou bien $R^2$ représente l'hydrogène et $R^3$ le chlore, le brome, l'iode ou un groupe cyano.

4. Composés selon l'une des rev. 1 à 3, caractérisés en ce que X représente un atome d'oxygène.

5. Composés selon l'une des rev. 1 à 4, caractérisés en ce que $R^1$ représente un groupe méthyle.

6. Le 7-chloro-5,6-dihydro-5-méthyl-6-oxo-4 H-imidazo[1,5-a][1,4]benzodiazépine-3-carboxylate de tert.-butyle.

7. Le 5,6-dihydro-7-iodo-5-méthyl-6-oxo-4 H-imidazo[1,5-a][1,4]benzodiazépine-3-carboxylate de tert.-butyle.

8. Le 7-bromo-5,6-dihydro-5-méthyl-6-oxo-4 H-imidazo[1,5-a][1,4]benzodiazépine-3-carboxylate de tert.-butyle.

9. Le 7-cyano-5,6-dihydro-5-méthyl-6-oxo-4 H-imidazo[1,5-a][1,4]benzodiazépine-3-carboxylate de tert.-butyle, le 5,6-dihydro-5-méthyl-6-oxo-4 H-imidazo[1,5-a][1,4]benzodiazépine-3-carboxylate de tert.-butyle, le 5,6-dihydro-5-méthyl-6-thioxo-4 H-imidazo[1,5-a][1,4]benzodiazépine-3-carboxylate de tert.-butyle, et le 8-fluoro-5,6-dihydro-5-méthyl-6-oxo-4 H-imidazo[1,5-a][1,4]benzodiazépine-3-carboxylate de tert.-butyle.

10. Composés selon l'une des rev. 1 à 9, en tant que substances actives pharmaceutiques.

11. Substances actives pharmaceutiques selon la rev. 10, antagonisant les propriétés de sédation centrale, de relaxation musculaire, d'ataxie, d'hypotension et de dépression respiratoire des 1,4-benzodiazépines à effet tranquillisant.

12. Procédé de préparation des composés selon l'une des rev. 1 à 9, caractérisé en ce que:

a) on fait réagir un composé de formule générale

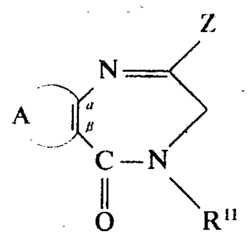

(II)

dans laquelle $R^{11}$ représente un groupe alkyle en C 1—C 7 et Z un groupe éliminable, A ayant la signification indiquée dans la rev. 1,
en présence d'une base avec l'ester isocyanacétique de formule

$$CN—CH_2—COOC(CH_3)_3 \qquad \text{(III)}$$

ou bien

b) dans un composé de formule générale

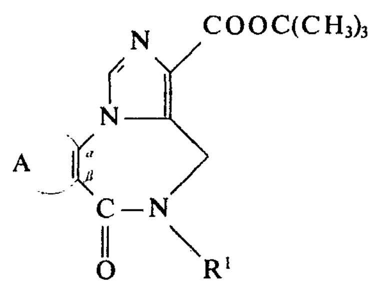

(Ia)

dans laquelle A et $R^1$ ont les significations indiquées dans la rev. 1,
on convertit le groupe carbonyle en groupe thiocarbonyle, ou bien

c) on convertit un acide carboxylique de formule générale

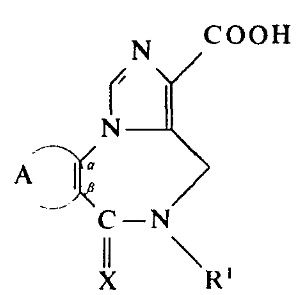

(IV)

dans laquelle A, $R^1$ et X ont les significations indiquées dans la rev. 1, en l'ester tert.-butylique correspondant, ou bien

d) on introduit le substituant voulu sur le groupe amino secondaire d'un composé de formule générale

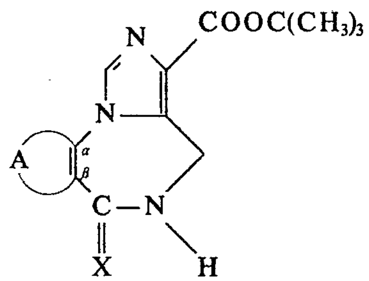

(Ib)

dans laquelle A et X ont les significations indiquées dans la rev. 1, ou bien

e) dans un composé de formule générale

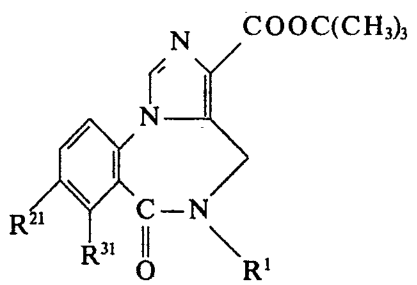

(Ic)

dans laquelle l'un des symboles $R^{21}$ et $R^{31}$ représente un halogène et l'autre l'hydrogène, $R^1$ ayant signification indiquée dans la rev. 1,
on remplace l'atome d'halogène par le groupe cyano, et

f) si on le désire, on convertit un composé obtenu, répondant à la formule générale I, en un sel formé par addition avec un acide acceptable pour l'usage pharmaceutique.

13. Médicaments contenant un composé selon l'une des rev. 1 à 9.

14. Médicaments selon la rev. 13, antagonisant les propriétés de sédation centrale, de relaxation musculaire, d'ataxie, d'hypotension et de dépression respiratoire des 1,4-benzodiazépines à effet tranquilli sant.

15. Médicament schistosomicide actif, contenant un composé selon l'une des rev. 1 à 9 et de la (+)-5-(o-chlorophényl)-1,3-dihydro-3-méthyl-7-nitro-2 H-1,4-benzodiazépine-2-one.

**Revendications pour l'Etat contractant: AT**

1. Procédé de préparation des imidazodiazépines de formule générale

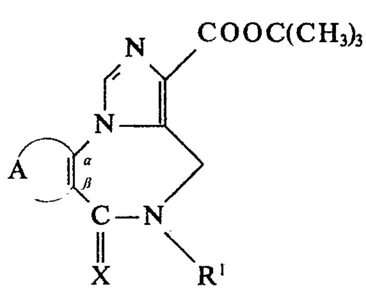

(I)

dans laquelle A forme avec les deux atomes de carbone marqués $\alpha$ et $\beta$ le groupe

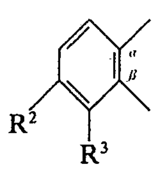

ou

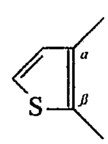

(a) (b)

X représente un atome d'oxygène ou de soufre et $R^1$ représente l'hydrogène ou un groupe alkyle en C 1—C 7, et ou bien $R^2$ représente l'hydrogène, un groupe trifluorométhyle, un halogène, un groupe cyano ou nitro et $R^3$ représente l'hydrogène, ou bien $R^2$ représente l'hydrogène et $R^3$ un groupe trifluorométhyle, un halogène, un groupe cyano, nitro ou alkyle en C 1—C 7, et de leurs sels formés par addition avec des acides acceptables pour l'usage pharmaceutique, caractérisé en ce que

a) on fait réagir un composé de formule générale

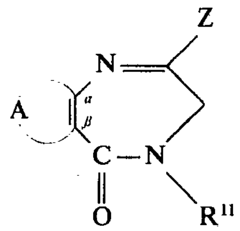

(II)

dans laquelle $R^{11}$ représente un groupe alkyle en C 1—C 7 et Z un groupe éliminable, A ayant la signification indiquée ci-dessus, en présence d'une base, avec l'ester isocyanacétique de formule

$$CN—CH_2—COOC(CH_3)_3 \quad (III)$$

ou bien

b) dans un composé de formule générale

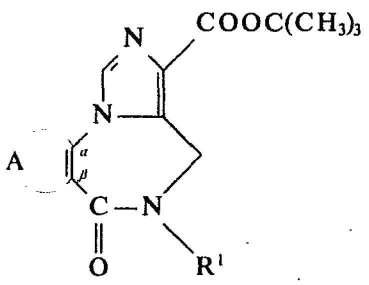

(Ia)

dans laquelle A et $R^1$ ont les significations indiquées ci-dessus, on convertit le groupe carbonyle en groupe thiocarbonyle, ou bien

c) on convertit un acide carboxylique de formule générale

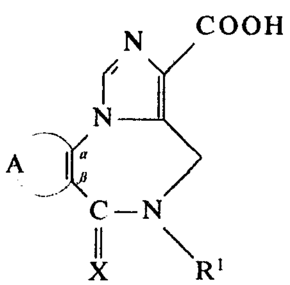

(IV)

dans laquelle A, $R^1$ et X ont les significations indiquées ci-dessus, en l'ester tert-butylique correspondant, ou bien

d) on introduit le substituant voulu sur le groupe amino secondaire d'un composé de formule générale

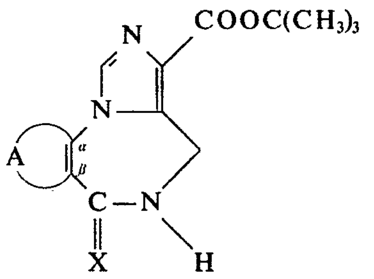

(Ib)

dans laquelle A et X ont les significations indiquées ci-dessus, ou bien

e) dans un composé de formule générale

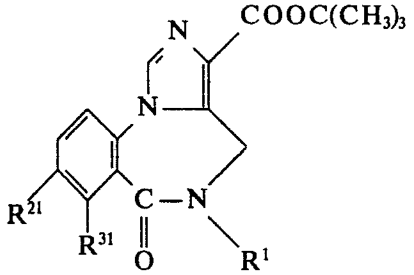

(Ic)

dans laquelle l'un des symboles $R^{21}$ et $R^{31}$ représente un halogène et l'autre l'hydrogène, $R^1$ ayant signification indiquée ci-dessus,

on remplace l'atome d'halogène par le groupe cyano, et

f) si on le désire, on convertit un composé obtenu de formule générale I en un sel formé par addition avec un acide acceptable pour l'usage pharmaceutique.

2. Procédé selon la revendication 1, caractérisé en ce que A représente le groupe (a) défini dans la revendication 1.

3. Procédé selon la revendication 2, caractérisé en ce que, ou bien $R^2$ représente l'hydrogène ou le fluor et $R^3$ l'hydrogène, ou bien $R^2$ représente l'hydrogène et $R^3$ le chlore, le brome, l'iode ou un groupe cyano.

4. Procédé selon l'une des revendications 1 à 3, caractérisé en ce que X représente un atome d'oxygène.

5. Procédé selon l'une des revendications 1 à 4, caractérisé en ce que $R^1$ représente le groupe méthyle.

6. Procédé selon la revendication 1, caractérisé en ce que l'on prépare le 7-chloro-5,6-dihydro-5-méthyl-6-oxo-4 H-imidazo[1,5-a][1,4]benzodiazépine-3-carboxylate de tert-butyle.

7. Procédé selon la revendication 1, caractérisé en ce que l'on prépare le 5,6-dihydro-7-iodo-5-méthyl-6-oxo-4 H-imidazo[1,5-a][1,4]benzodiazépine-3-carboxylate de tert-butyle.

8. Procédé selon la revendication 1, caractérisé en ce que l'on prépare le 7-bromo-5,6-dihydro-5-méthyl-6-oxo-4 H-imidazo[1,5-a][1,4]benzodiazépine-3-carboxylate de tert-butyle.